# EUROPÄISCHE PATENTSCHRIFT

(11) **EP 2 823 309 B1**
(45) Veröffentlichungstag und Bekanntmachung des Hinweises auf die Patenterteilung: **02.03.2016**
(21) Anmeldenummer: 13707408.4
(22) Anmeldetag: 06.03.2013
(51) Int. Cl.: G01N 33/558

(54) **VERFAHREN UND VORRICHTUNG ZUM NACHWEIS VON ANALYTEN**
METHOD AND DEVICE FOR DETECTING ANALYTES
PROCÉDÉ ET DISPOSITIF DESTINÉS À LA VÉRIFICATION D'ANALYTES

(30) Priorität: 06.03.2012 EP 12158247
(43) Veröffentlichungstag der Anmeldung: 14.01.2015
(73) Patentinhaber: Securetec Detektions-Systeme AG, 85579 Neubiberg (DE)
(72) Erfinder: STADTHAGEN, Torsten, 81475 München (DE); SCHWIEGER, Frank, 38126 Braunschweig (DE); KLAUS, Sebastian, 82061 Neuried (DE)
(74) Vertreter: Weickmann & Weickmann
(86) Internationale Anmeldenummer: PCT/EP2013/054499
(87) Internationale Veröffentlichungsnummer: WO 2013/131955

(56) Entgegenhaltungen:
- EP-A1- 1 700 618
- EP-A1- 2 381 258
- US-A1- 2005 084 855
- US-A1- 2005 175 992
- L. Zhan ET AL: "Effects of Xylitol Wipes on Cariogenic Bacteria and Caries in Young Children", Journal of Dental Research, vol. 91, no. 7 Suppl, 14 June 2012 (2012-06-14), pages S85-S90, XP055114365, ISSN: 0022-0345, DOI: 10.1177/0022034511434354
- G L Smith ET AL: "Survival of gram positive anaerobic cocci on swabs and their isolation from the mouth and vagina.", Journal of Clinical Pathology, vol. 39, no. 1, 1 January 1986 (1986-01-01), pages 93-98, XP055114364, ISSN: 0021-9746, DOI: 10.1136/jcp.39.1.93

## Beschreibung

Die vorliegende Erfindung betrifft ein Verfahren zur Bestimmung von Analyten, sowie eine hierzu geeignete Vorrichtung.

Immunoassays stellen einen wichtigen Bestandteil klinisch relevanter Analyseverfahren dar, welche auf der Bildung von Immunkomplexen aus Antigenen und Antikörpern basieren und dem Nachweis von Analyten, beispielsweise im Bereich der Medizin-, Umwelt-, Lebensmittel- und Agraranalytik, in flüssigen Proben dienen. Immunoassays bieten aufgrund der biophysikalischen und biochemischen Eigenschaften der Antigen-Antikörper-Bindung im Allgemeinen eine hohe Spezifität und Sensitivität bei vergleichsweise geringem apparativen und wirtschaftlichen Aufwand und weisen insofern signifikante Vorteile gegenüber alternativen Nachweisverfahren auf.

Ein weiteres in der Praxis bedeutendes Anwendungsgebiet von Immunosassays stellt der Nachweis von Drogen in Körperflüssigkeiten oder auf mit Drogen kontaminierten Oberflächen dar. Im Rahmen derartiger Tests wird üblicherweise eine Probe des Analyten mit einem geeigneten Probennahmeelement von einer Oberfläche aufgenommen, das mit dem Analyten benetzte Probennahmeelement mit einem Teststreifen in Kontakt gebracht, und der aus dem Probennahmeelement herausgelöste und auf den Teststreifen transferierte Analyt mittels einer immunologischen Nachweisreaktion detektiert.

Ein Aspekt, welcher speziell beim Nachweis von Drogen (z.B. Amphetamine, Metamphetamine, Cannabis, Kokain, Heroin) eine herausragende Bedeutung besitzt, ist die Spezifität, Sensitivität und Schnelligkeit der verwendeten Tests. Hierbei besteht zum einen das Bedürfnis hinsichtlich hochsensitiver Nachweisverfahren, um selbst im Falle kleiner Probenvolumina die Anwesenheit von Drogen zuverlässig und schnell nachweisen zu können. Andererseits sollten die Testformate auch eine hohe Spezifität für die jeweils nachzuweisende Substanz aufweisen, um falschpositive Messergebnisse auszuschließen und insofern eine verlässliche Aussage darüber zu liefern, um welche konkrete Droge es sich bei der getesten Substanz handelt.

EP 0 699 906 A2 offenbart einen Drogenschnelltest, welcher als Oberflächen-, Schweiß- bzw. Speicheltest unter der Bezeichnung DrugWipe^{®} (Firma Securetec Detektionssysteme AG) kommerziell erhältlich ist. Der Test bedient sich eines im wesentlichen aus Kunststoff bestehenden Wischelements ("Wischer") mit aufgeschweißtem Vlies, mittels welchem eine Probe des Analyten (z.B. von einer Oberfläche oder aus einer Lösung) genommen und anschließend direkt auf einen immunchromatographischen Teststreifen, welcher in einem Einweggehäuse gelagert ist, übertragen wird ("Lateral Flow"-Technologie). US 2005175992 offenbart Verfahren, Testelemente und Kits zur Bestimmung eines Analyten. EP 2381258 offenbart ein ähnliches Testelement. US2005/084855 offenbart Übertragungsreagenzien wie BSA, die freie Bindungsstellen auf dem Wischelement blockieren. Xylitol Wipes sind bekannt aus Zhan et al. Journal of Dental Research, 2012, Vol.91, Nr. 7 Suppl. 1; S85-S90.

Durch Eintauchen des Teststreifens in eine wässrige Lösung (Wasser oder Puffer mit verschiedenen Reagenzien) wird die Chromatographie gestartet, wobei das Ergebnis der Bestimmung mit dem Auge oder unter Verwendung einer geeigneten Messvorrichtung ausgelesen werden kann. Der Nachweis des Analyten erfolgt dadurch, dass an Drogenmoleküle (Antigene) gebundene Antikörper an einer Testlinie abbinden und aufgrund ihrer Goldmarkierung eine farbige Linie bilden, welche im Auslesefenster optisch detektiert werden kann. Antikörper, welche nicht mit Drogenmolekülen beladen sind, werden vor Erreichen der Testlinie mittels Haptenen, die auf dem Teststreifen in immobilisierter Form vorliegen, abgefangen.

Im Vergleich zu allen anderen auf dem Markt befindlichen Drogenschnelltests ist DrugWipe^{®} das einzige Produkt, welches bei Vorhandensein eines bestimmten Analyten in einer Probe eine farbige Linie ausbildet und insofern eine sogenannte Positivanzeige aufweist. Alle übrigen kommerziellen Produkte verfügen über eine Negativanzeige, bei der das Nicht-Erscheinen einer Linie als positiver Nachweis für den jeweiligen Analyten gewertet wird. Ein weiterer Vorteil des DrugWipe^{®} ist das geringe Probenvolumen, welches zum Nachweis von Drogen und anderen Substanzen benötigt wird. Während im Falle von DrugWipe^{®} ein Probenvolumen von etwa 1-50 µl ausreichend ist, erfordern andere kommerzielle Tests ein Probenvolumen von mindestens 100 µl bis hin zu mehreren Millilitern.

Das geringe Probenvolumen, welches zur Durchführung des DrugWipe^{®}-Drogenschnelltests benötigt wird, stellt einen entscheidenden Vorteil gegenüber anderen kommerziell verfügbaren Testsystemen dar, da Drogenkonsumenten aufgrund der physiologischen Wirkung der Drogen häufig einen sehr trockenen Mund haben und nur wenig Speichel aktiv hervorrufen können. Somit stehen im Falle von Drogenkonsumenten üblicherweise nur sehr geringe Probenvolumina zur Verfügung, welche mit Ausnahme des DrugWipe^{®}-Drogenschnelltests allgemein nicht ausreichen, um ein vertrauenswürdiges bzw. fehlerfreies Ergebnis zu erhalten. Sofern die Durchführung eines Drogenschnelltests in solchen Fällen überhaupt möglich ist, dauert die Probennahme häufig mehrere Minuten, was dem Probanden letztlich nicht zumutbar ist.

Der von der Firma Varian entwickelte Schnelltest OraLab^{®} 6 dient zum Nachweis von Drogen aus Speichelproben und basiert ebenfalls auf der Lateral-Flow-Technologie. Während die Spezifität des Tests bei etwa 90-100% liegt, beträgt die Sensitivität bei Amphetaminen und Opiaten lediglich zwischen 50 und 90%, bei Δ⁹-Tetrahydrocannabinol und Kokain zum Teil sogar deutlich unter 50% (siehe DRUID-Studie, veröffentlicht auf der TIAFT 2009 in Genf). Ein großer Nachteil dieses Tests liegt weiterhin darin, dass relativ große Probenvolumina benötigt werden, welche bei akuten Drogenkonsumenten oft nicht zur Verfügung stehen.

Der Schnelltest DrugCheck^{®} 5000 der Firma Dräger, welcher ebenfalls auf der Lateral-Flow-Technologie basiert, stellt gemäß den Daten der DRUID-Studie (TIAFT 2009, Genf) ein zuverlässiges Testsystem zum Nachweis von Drogen dar. Allerdings besitzt dieses Testformat den signifikanten Nachteil, dass die Ergebnisse auf dem Teststreifen nur mittels eines Auslesegeräts, welches in der Anschaffung sehr teuer und für den harten Feldeinsatz im Freien nur teilweise geeignet ist, ausgewertet werden können. Insofern ergeben sich für diesen Test erhebliche Probleme in Bezug auf Wirtschaftlichkeit und einfache Handhabbarkeit.

Der von der Firma Mavand angebotene Testkit Rapid STAT^{®} stellt einen weiteren kommerziell erhältlichen Drogenschnelltest dar, bei welchem eine mit Puffer verdünnte Speichelprobe auf einem immunchromatographischen Teststreifen mit markierten Bindungspartnern inkubiert wird und welcher laut Herstellerangaben einen Nachweis bis zu einer unteren Grenze von 15 ng/ml an Δ⁹-Tetrahydrocannabinol ermöglicht.

Ein signifikanter Nachteil dieses Tests ist dessen äußerst komplexe und umständliche Handhabung, welche ihn gerade für eine Anwendung bei der Verkehrspolizei ungeeignet macht, sowie die vergleichsweise geringe Spezifität für Δ⁹-Tetrahydrocannabinol von 80-90% (siehe DRUID-Studie, TIAFT 2009, Genf). Laut einer Studie der Universität Mainz liegt die Rate von falsch-positiven Testergebnissen für Δ⁹-Tetrahydrocannabinol bei mehr als 10% und die Gesamtspezifität bei 84% (veröffentlicht auf der GTFCH 2009, Mosbach, http://www.gtfch.org/cms/images/stories/media/tk/tk76_2/ abstractsposter.pdf).

US 2005/0084855 A1 offenbart ein Verfahren zur Bestimmung eines Analyten in einer Probe, welches das Inkontaktbringen der Probe mit einem zur Bindung des Analyten befähigten Träger, das Inkontaktbringen des analythaltigen Trägers mit einem Stempel, der eine zur Bindung des Analyten geeignete Sonde beinhaltet, sowie das Überprüfen der an den Träger gebundenen Menge an Sonde umfasst. Ein wesentlicher Nachteil des Nachweisverfahrens besteht darin, dass der Stempel nicht zur Aufnahme einer Probe des Analyten von einer zu untersuchenden Oberfläche geeignet ist und darüber hinaus technische Hilfsmittel (wie beispielsweise ein Fluoreszenz-Scanner) zum Auswerten der Ergebnisse benötigt werden.

WO 2005/075982 A2 und US 2005/0175992 A1 offenbaren jeweils ein Verfahren zur Bestimmung eines Analyten ausgewählt aus Pathogenen oder/und Allergie-assoziierten Substanzen in einem Körperfluid. Im Rahmen des Verfahrens wird das Körperfluid mittels eines separaten oder in ein Testelement integrierten Wischelements aufgenommen und dieses für einen Zeitraum von vorzugsweise 0.1 bis 10 Sekunden mit dem Testelement in Kontakt gebracht, wobei zumindest ein Teil der Probe von dem Wischelement auf das Testelement übertragen wird und der Analyt, nach Start der Chromatographie und Bindung an einen analytspezifischen Bindungspartner, analysiert wird. Ferner wird ein Kit zur Detektion von Adenoviren beschrieben, welcher einen chromatografischen Teststreifen in Kombination mit einem Wischelement umfasst.

Ein entscheidender Nachteil des in WO 2005/075982 A2 und US 2005/0175992 A1 beschriebenen Nachweisverfahrens besteht darin, dass vor dem Beginn der Chromatographie kein direkter Kontakt zwischen dem Analyten und dem analytspezifischen Bindungspartner realisiert wird, womit die Ausbildung eines Komplexes aus Analyt und analytspezifischem Bindungspartner erst im Zuge der Chromatographie vollzogen werden kann. Darüber hinaus wird das den Analyten enthaltende Körperfluid vor der im Testelement erfolgenden Bindung an den analytspezifischen Bindungspartner in keinster Form aufbereitet, beispielsweise durch chemische oder/und mechanische Vorbehandlung der Probe, womit de facto keine optimale Bindung zwischen Analyt und analytspezifischem Bindungspartner erzielt werden kann.

Allerdings unterliegen insbesondere biologische Proben mit komplexer chemischer Zusammensetzung (z.B. Blut, Speichel oder Schweiß) einer personenabhängigen Schwankungsbreite beispielsweise in Bezug auf Konsistenz, Viskosität, Salzgehalt oder/und Proteingehalt und bieten eine Vielzahl an unspezifischen Bindungsstellen für den Analyten. Wird eine biologische Probe vor dem Beginn der Chromatographie nicht vorbehandelt, bedingt dies schlecht reproduzierbare Ergebnisse sowie Einbußen im Hinblick auf die Sensitivität des Verfahrens, da der Analyt infolge der Bindung an unspezifische Bindungsstellen in der Probenmatrix nicht mehr für den eigentlichen Analytnachweis zur Verfügung steht.

EP 0 811 842 A2 und EP 0 699 906 A2 offenbaren ein Verfahren zum Nachweis eines Analyten in einer Körperflüssigkeit bzw. auf einer kontaminierten Oberfläche. Zur Durchführung des Verfahrens wird hierbei jeweils ein Testkit verwendet, welcher einen Teststreifen aus einem oder mehreren kapillaraktiven chromatografiefähigen Materialien, ein Probennahmeelement separat zur Teststreifenoberfläche, sowie eine Andrückvorrichtung zum Inkontaktbringen von Teststreifenoberfläche und Probennahmeelement umfasst, wobei das Probennahmeelement zwecks besserer Aufnahme des Analyten mit einem wässrigen Puffer, einem Detergenz oder/und einem organischen Lösungsmittel angefeuchtet sein kann.

EP 2 381 258 A1 offenbart ein Verfahren zur Bestimmung eines Analyten, welches das Bereitstellen einer mikrofluidischen Analysevorrichtung, das Aufnehmen einer den Analyten enthaltenden Probe von einer Probenoberfläche mit einem Wischelement, das Einbringen des analythaltigen Wischelements in die mikrofluidische Analysevorrichtung, das Eluieren des Analyten von dem Wischelement innerhalb der mikrofluidischen Analysevorrichtung mit einem Elutionsmittel, sowie das Bestimmen des Analyten auf einem in der mikrofluidischen Analysevorrichtung gelagerten Testelement umfasst. In einer bevorzugten Variante umfasst das Wischelement ein Übertragungsreagenz, welches mindestens ein Protein, mindestens ein Kohlenhydrat, mindestens einen Zuckeralkohol oder/und mindestens ein Salz enthält.

WO 2005/121793 A2 offenbart ein Verfahren zum Nachweis eines Methamphetamins in einer flüssigen Probe. Zur Durchführung des Verfahrens wird vorzugsweise ein Kit verwendet, welcher einen chromatografischen Teststreifen und gegebenenfalls ein Probennahmeelement umfasst. Der Teststreifen umfasst seinerseits (a) ein trockenes poröses Material, auf welchem ein Pseudoephedrin/Träger-Konjugat oder ein Antikörper, der sowohl an das Methamphetamin als auch an das Konjugat binden kann, in einer Detektionszone immobilisiert ist, und (b) eine hiervon getrennte Markerfreisetzungszone, welche entweder den Antikörper in markierter Form, sofern die Detektionszone immobilisiertes Pseudoephedrin/Träger-Konjugat enthält, oder detektierbares Pseudoephedrin/Träger-Konjugat, sofern die Detektionszone immobilisierten Antikörper enthält, in die Flüssigkeit freisetzen kann.

Auch die in EP 0 699 906 A2, EP 0 811 842 A2, EP 2 381 258 A1 und WO 2005/121793 A2 beschriebenen Nachweisverfahren haben den Nachteil, dass keine Vorbehandlung der Probe vor dem Beginn der Chromatographie vorgenommen wird oder/und keine direkte bzw. definierte Inkubation zwischen dem Analyten und dem analytspezifischen Bindungspartner stattfindet. Vielmehr kommen der Analyt und der analytspezifische Bindungspartner erst im Laufe der Chromatographie miteinander in Kontakt, wodurch keine optimale Bindung zwischen dem Analyten und dem analytspezifischen Bindungspartner realisiert werden kann und letztlich die Sensitivität und Spezifität des Verfahrens, insbesondere beim Nachweis von schlecht wasserlöslichen Analyten, negativ beeinflusst wird. Dies führt zumindest teilweise dazu, dass die gesetzlich geforderten Mindestnachweisgrenzen für Drogenmoleküle nicht erreicht werden.

Ausgehend von oben beschriebenen Testsystemen bestand die der vorliegenden Erfindung zugrunde liegende Aufgabe somit darin, ein Verfahren zur Bestimmung eines Analyten, insbesondere zur Bestimmung von Drogen, bereitzustellen, bei welchem die Nachteile des Standes der Technik zumindest teilweise beseitigt sind. Insbesondere sollte das Verfahren für alle zu testenden Analyten eine hohe Sensitivität und Spezifität aufweisen, leicht handhabbar sein und eine schnelle Bestimmung der Analyten ohne den Einsatz zusätzlicher technischer Hilfsmittel ermöglichen.

Diese Aufgabe wird erfindungsgemäß gelöst durch ein Verfahren zur Bestimmung eines Analyten, umfassend die Schritte:
(a) Bereitstellen eines Testelements, umfassend
   (i) einen ersten Bereich, welcher zur Aufnahme von Elutionsmittel ausgebildet ist,
   (ii) einen zweiten Bereich, welcher zur Applikation einer den Analyten enthaltenden Probe ausgebildet ist und einen für den Analyten spezifischen Bindungspartner umfasst,
   (iii) einen dritten Bereich, welcher zur optischen Detektion des Analyten ausgebildet ist,
   (iv) einen vierten Bereich, welcher zur Aufnahme von überschüssigem Elutionsmittel ausgebildet ist, und
   (v) gegebenenfalls ein Gehäuse,
(b) Aufnehmen einer den Analyten enthaltenden Probe von einer Probenoberfläche mit einem Wischelement,
(c) Inkontaktbringen des Wischelements mit dem zweiten Bereich des Testelements für einen Zeitraum von mindestens 10 Sekunden, wobei zumindest ein Teil der Probe von dem Wischelement auf das Testelement übertragen wird und eine Inkubation des Analyten mit dem analytspezifischen Bindungspartner erfolgt,
(d) Inkontaktbringen des ersten Bereichs des inkubierten Testelements mit einem Elutionsmittel, und
(e) Bestimmen des Vorhandenseins oder/und der Menge des Analyten, wobei das Testelement oder/und das Wischelement ein Analyt-Übertragungsreagenz umfasst, welches mindestens eine analytunspezifische Substanz ausgewählt aus der Gruppe bestehend aus einem Protein, einem Proteingemisch, einem Kohlenhydrat und einem Zuckeralkohol enthält.

Überraschenderweise wurde im Rahmen der vorliegenden Erfindung festgestellt, dass mittels des erfindungsgemäßen Verfahrens Analyten einfach und reproduzierbar mit hoher Sensitivität und Spezifität nachgewiesen werden können, ohne dass hierzu große Probenmengen des Analyten und komplexe technische Hilfsmittel beispielsweise zum Auslesen der Messergebnisse erforderlich sind.

Der erste Schritt des erfindungsgemäßen Verfahrens erfordert die Bereitstellung eines für die Bestimmung des Analyten geeigneten Testelements, welches einen für den Analyten spezifischen Bindungspartner umfasst. Die zu diesem Zweck verwendeten Testelemente umfassen grundsätzlich jede dem Fachmann geläufige physikalische Form, welche für die Bestimmung des Vorhandenseins oder/und der Menge eines Analyten in einer Probe geeignet ist. Hierbei ist das Testelement vorzugsweise derart ausgebildet, dass es bei Anwesenheit des zu bestimmenden Analyten ein optisch detektierbares Signal erzeugt, welches eine qualitative oder/und quantitative Bestimmung des Analyten ermöglicht. Beispiele für Testelemente im Sinne der vorliegenden Erfindung umfassen u.a. Teststreifen, Testbänder und Testpads, auf die der Analyt beispielsweise in Form einer wässrigen oder nichtwässrigen Lösung aufgebracht werden kann.

In einer bevorzugten Ausführungsform handelt es sich bei dem Testelement um einen chromatographischen Teststreifen, welcher in einer Variante der Erfindung aus einem einzigen chromatographiefähigen, gegebenenfalls streifenförmigen Material gebildet sein kann. Vorzugsweise umfasst der chromatographische Teststreifen allerdings mehrere, auf einer Trägerschicht überlappend angeordnete kapillaraktive Flächen aus gleichen oder unterschiedlichen chromatographischen Materialien, welche in fluider Kommunikation miteinander stehen und auf diese Weise eine Transportstrecke bilden, entlang derer ein Fluid, durch Kapillarkräfte getrieben, durch sämtliche Bereiche des Testelements strömen kann. Als chromatographisches Material kann dabei jedes bekannte flüssigkeitsabsorbierende, poröse oder kapillaraktive Material verwendet werden, wie z.B. Cellulose und Derivate hiervon, Glasfasern, sowie Vliese und Gewebe aus künstlichen oder natürlichen Materialien. Chromatographische Teststreifen, welche im Rahmen der vorliegenden Erfindung Anwendung finden können, sind beispielsweise in EP 0 699 906 A2 beschrieben, auf deren Offenbarung hiermit ausdrücklich Bezug genommen wird.

Der analytspezifische Bindungspartner kann eine beliebige chemische Substanz sein, welche an den zu testenden Analyten bindet, jedoch keinerlei Bindungen zu anderen in der Probe oder/und im Testelement eventuell vorhandenen Stoffen ausbildet. Chemische Substanzen, welche diesem Anforderungsprofil genügen, sind dem Fachmann allgemein bekannt oder können entsprechend den Anforderungen an das jeweilige Testelement mittels Routineexperimenten und unter Anwendung bekannter Techniken hergestellt werden. Vorzugsweise ist der analytspezifische Bindungspartner in den hierin beschriebenen Testelementen wanderungsfähig und kann beispielsweise mittels eines geeignet positionierten Abfangreagenzes an einer vorbestimmten Position auf dem Testelement immobilisiert werden. Analytspezifische Bindungspartner, deren Einsatz sich als besonders vorteilhaft erwiesen hat, sind beispielsweise in EP 1 579 222 B1 beschrieben, auf deren Offenbarung ausdrücklich Bezug genommen wird.

In einer bevorzugten Ausführungsform handelt es sich bei dem analytspezifischen Bindungspartner um einen Antikörper oder ein funktionelles Antikörperfragment, welcher gegebenenfalls zusätzlich eine Bindungsstelle oder mehrere Bindungsstellen für ein Abfangreagenz, wie es oben beschrieben ist, aufweisen können. Der Begriff "funktionelles Antikörperfragment", wie er im Rahmen der vorliegenden Anmeldung verwendet wird, bezeichnet hierbei ein Antikörperfragment, das die ihm zugedachte Funktion einer spezifischen Bindung an den Analyten erfüllen kann. Ein "nicht-funktionelles Antikörperfragment" bezeichnet demgegenüber ein Antikörperfragment, welches keine Bindung oder keine spezifische Bindung mit dem Analyten ausbildet und insofern die ihm zugedachte Funktion einer spezifischen Bindung an den Analyten nicht erfüllt.

Um den Nachweis des Analyten mittels der hierin beschriebenen Testelemente zu erleichtern, kann der analytspezifische Bindungspartner gegebenenfalls eine detektierbare Markierung, wie beispielsweise eine Enzymmarkierung, Farbstoffmarkierung, Fluoreszenzmarkierung, Metallmarkierung oder Partikelmarkierung, umfassen. Für die Zwecke der vorliegenden Erfindung hat sich die Verwendung analytspezifischer Bindungspartner, welche eine optisch detektierbare Markierung, insbesondere eine Metallmarkierung, umfassen, als vorteilhaft erwiesen. Besonders bevorzugt handelt es sich bei der Markierung um eine Goldmarkierung, welche den Vorteil besitzt, dass das Testergebnis vom Anwender direkt optisch-visuell erfasst und ausgewertet werden kann. Techniken, mittels welcher oben beschriebene Markierungen in ein zu markierendes Molekül eingeführt werden können, sind dem Fachmann bekannt und werden insofern nicht näher erläutert.

Zur Bestimmung des Analyten unter Verwendung der hierin beschriebenen Testelemente wird eine den Analyten enthaltende Probe in einem weiteren Schritt des erfindungsgemäßen Verfahrens mittels eines geeigneten Wischelements von einer zu untersuchenden Oberfläche (z.B. Zunge, Haut, sonstige Oberfläche) aufgenommen. Als Wischelement kann dabei ein beliebiges Element verwendet werden, welches eine Probe des Analyten aufzunehmen vermag und einen nachfolgenden Transfer der Probe auf das Testelement, beispielsweise unter Ausnutzung von Kapillareffekten, ermöglicht. Das Wischelement weist eine oder mehrere (voneinander unabhängige) Wischflächen auf, wobei im Falle mehrerer Wischflächen Wischelemente mit 2, 3 oder 4 Wischflächen bevorzugt sind. Sofern ein Wischelement mit mehreren Wischflächen zum Einsatz gelangt, kommt beim späteren Zusammenführen einer Testvorrichtung, welche mehrere (voneinander unabhängige) Testelemente umfasst, und dem Wischelement vorzugsweise jeweils ein Testelement mit einer Wischfläche des Wischelements in Kontakt.

Die mindestens eine Wischfläche, welche vorzugsweise mit einer Oberfläche des Wischelements verschweißt ist, kann grundsätzlich aus einem beliebigen Material bestehen, das dem Fachmann für die Zwecke der vorliegenden Erfindung nützlich erscheint und sowohl eine Anreicherung des Analyten auf der Wischfläche als auch eine spätere Übertragung des Analyten auf das Testelement ermöglicht. Als zweckmäßig haben sich hierbei speziell saugfähige Materialien, insbesondere Gewebe, Vliese oder/und poröse Matrices (z.B. Membranen und Schwämme), erwiesen. Besonders bevorzugt werden im Rahmen der Erfindung Vliese, insbesondere Vliese auf Basis von Cellulose-, Polyester- oder/und Glasfasern, eingesetzt, wobei die Fasern gegebenenfalls mit Hilfe eines organischen Bindemittels zusammengehalten werden können. Geeignete Vliese bzw. Fasern sind beispielsweise in DE 38 02 366 A1 und EP 0 699 906 A2 beschrieben, auf deren Offenbarung ausdrücklich Bezug genommen wird.

Was die äußere Ausgestaltung der Wischfläche(n) betrifft, so bestehen grundsätzlich keinerlei Einschränkungen hinsichtlich der Dicke, Dimension und Geometrie. Die Dicke der Wischfläche(n) bzw. des hierfür verwendeten Materials ist für die Zwecke der vorliegenden Erfindung von untergeordneter Bedeutung und liegt üblicherweise in einem Bereich von 0.1 bis 3 mm. Die Dimension der Wischfläche(n) ist vorteilhafterweise der Dimension des Testelements angepasst, d.h. die Breite der Wischfläche(n) sollte die Breite des Testelements weder übersteigen noch unterbieten. Bevorzugte Dimensionen der Wischfläche(n) liegen bezüglich der Länge im Bereich von 0.3 bis 2 cm, bezüglich der Breite im Bereich von 0.3 bis 1 cm. Die Geometrie der Wischfläche(n) kann den jeweiligen Anforderungen an die zu untersuchende Oberfläche angepasst werden, wobei eine dreieckige, viereckige (z.B. quadratische, rechteckige, rautenförmige) oder rollenförmige Ausgestaltung der Wischfläche(n) als besonders vorteilhaft angesehen wird. Eine rollenförmige Ausgestaltung der Wischfläche(n) hat dabei den Vorteil, dass durch die große Oberfläche der Wischfläche(n) ein besonders guter Kontakt zwischen dem Wischelement und dem zweiten Bereich des Testelements realisiert wird und dementsprechend eine Erhöhung der Sensitivität des Verfahrens erzielt werden kann.

Um eine besonders hohe Sensitivität und Spezifität bei der Bestimmung des Analyten zu gewährleisten, sieht das erfindungsgemäße Verfahren vor, dass das Testelement oder/und das Wischelement ein Analyt-Übertragungsreagenz umfasst. In einer Ausführungsform der Erfindung umfasst das zur Bestimmung des Analyten eingesetzte Testelement das Analyt-Übertragungsreagenz, während in einer anderen Ausführungsform das Wischelement das Analyt-Übertragungsreagenz beinhaltet. Besonders bevorzugt umfassen indessen sowohl das Testelement als auch das Wischelement ein Analyt-Übertragungsreagenz, wie es nachfolgend im Detail definiert ist.

Das Analyt-Übertragungsreagenz enthält mindestens eine analytunspezifische Substanz ausgewählt aus der Gruppe bestehend aus einem Protein, einem Proteingemisch, einem Kohlenhydrat und einem Zuckeralkohol, wobei der Begriff "analytunspezifische Substanz" eine chemische Substanz bezeichnet, welche nicht ausschließlich an den zu testenden Analyten bindet, sondern auch Bindungen zu anderen in der Probe oder/und im Testelement eventuell vorhandenen Stoffen ausbilden kann. Die Konzentration an Protein, Proteingemisch, Kohlenhydrat bzw. Zuckeralkohol in dem Analyt-Übertragungsreagenz/den Analyt-Übertragungsreagenzien kann entsprechend den jeweiligen Anforderungen an das Testelement vom Fachmann angepasst werden, beträgt jedoch üblicherweise etwa 0.01 bis etwa 15 Gew.-%, bezogen auf das Gesamtgewicht des Analyt-Übertragungsreagenzes.

Sofern das Testelement das Analyt-Übertragungsreagenz umfasst, so ist es ferner als bevorzugt anzusehen, dass das Analyt-Übertragungsreagenz ein Kohlenhydrat oder/und einen Zuckeralkohol, insbesondere einen Zuckeralkohol, enthält. Demgegenüber umfasst das Wischelement vorzugsweise ein Analyt-Übertragungsreagenz, welches ein analytunspezifisches Protein oder/und ein analytunspezifisches Proteingemisch, insbesondere ein analytunspezifisches Protein, enthält. Als analytunspezifisches Protein wird in den hierin beschriebenen Testelementen oder/und Wischelementen bevorzugt ein Albumin, insbesondere Ovalbumin oder Rinderserumalbumin, eingesetzt, während als analytunspezifisches Proteingemisch beispielsweise Gelatine oder Magermilchpulver zur Anwendung gelangen kann.

Der Begriff "Kohlenhydrat", wie er in der vorliegenden Anmeldung verwendet wird, bezeichnet Monosaccharide und Oligosaccharide, insbesondere Disaccharide und Trisaccharide, der allgemeinen Summenformel CₙH₂ₙOₙ, welche jeweils natürlichen oder synthetischen Ursprungs sein können. Als Monosaccharide gelangen insbesondere natürlich vorkommende Tetrosen, Pentosen und Hexosen, wie beispielsweise Erythrose, Threose, Ribose, Arabinose, Lyxose, Xylose, Allose, Altrose, Galactose, Glucose, Gulose, Idose, Mannose, Talose und Fructose, die jeweils in der D-Form oder in der L-Form vorliegen können, zum Einsatz. Als Oligosaccharide können insbesondere natürlich vorkommende Disaccharide und Trisaccharide verwendet werden, wie beispielsweise Lactose, Maltose, Saccharose, Trehalose, Gentianose, Kestose und Raffinose. In einer besonders bevorzugten Ausführungsform der Erfindung handelt es sich bei dem Kohlenhydrat um eine Substanz ausgewählt aus der Gruppe bestehend aus Glucose, Lactose, Maltose, Mannose und Saccharose.

Der Begriff "Zuckeralkohol", wie er in der vorliegenden Anmeldung verwendet wird, bezeichnet Monosaccharid-Zuckeralkohole der allgemeinen Summenformel CₙH₂ₙ₊₂Oₙ und Disaccharid-Alkohole der allgemeinen Summenformel CₙH₂ₙOₙ₋₁, welche jeweils natürlichen oder synthetischen Ursprungs sein können. Bevorzugte Monosaccharid-Zuckeralkohole umfassen Glycerol, Erythritol, Threitol, Ribitol, Arabinitol, Xylitol, Allitol, Altritol, Galactitol, Glucitol, Iditol und Mannitol, die jeweils in der D-Form oder in der L-Form vorliegen können. Als Disaccharid-Zuckeralkohole können insbesondere Isomalt, Lactitol und Maltitol verwendet werden. In einer besonders bevorzugten Ausführungsform der Erfindung handelt es sich bei dem Zuckeralkohol um eine Substanz ausgewählt aus der Gruppe bestehend aus Glucitol, Glycerol, Lacitol, Mannitol und Xylitol.

Das Analyt-Übertragungsreagenz, welches den Transfer des Analyten von der zu untersuchenden Oberfläche auf das Wischelement oder/und die nachfolgende Übertragung des Analyten vom Wischelement auf das Testelement insbesondere durch Blockierung freier Bindungsstellen auf dem Wischelement oder/und durch Beeinflussung der Analyteigenschaften begünstigt, kann beispielsweise auf dem Testelement oder/und dem Wischelement, insbesondere im Bereich der Wischfläche(n), imprägniert sein. Techniken, welche zur Aufbringung des Analyt-Übertragungsreagenzes auf das Testelement oder/und das Wischelement verwendet werden können, sind dem Fachmann bekannt und können entsprechend den Anforderungen an das Testelement gezielt ausgewählt werden.

Um eine möglichst vollständige Aufnahme des Analyten zu gewährleisten, kann die mindestens eine Wischfläche des Wischelements im Falle der Beprobung trockener Oberflächen mit einer geringen Menge, beispielsweise mit etwa 1-100 µl, einer geeigneten Flüssigkeit angefeuchtet werden. Zu diesem Zweck kann die Flüssigkeit beispielsweise mittels einer Pipette, einer automatischen Dosiervorrichtung, oder eines die Flüssigkeit abgebenden Materials (z.B. einem mit der Flüssigkeit getränkten Schwamm) auf die Wischfläche(n) aufgebracht werden. Alternativ kann die Befeuchtung der Wischfläche(n) auch derart erfolgen, dass die mindestens eine Wischfläche die Flüssigkeit in mikroverkapselter oder blisterverpackter Form umfasst und die Flüssigkeit durch geeignete Maßnahmen, beispielsweise durch Andrücken der Wischfläche(n) auf die zu untersuchende Oberfläche, freigesetzt wird. Flüssigkeiten, welche für eine Anfeuchtung der Wischfläche(n) geeignet sind, umfassen insbesondere Wasser und wässrige Pufferlösungen, die gegebenenfalls weitere Substanzen wie beispielsweise ein Detergenz oder/und ein organisches Lösungsmittel, wie jeweils oben beschrieben, enthalten können. Sofern das Wischelement mit feuchten Oberflächen oder flüssigen Proben in Kontakt gebracht wird, ist ein vorheriges Anfeuchten der Wischfläche(n) in der Regel nicht erforderlich.

Nach Aufnahme der Probe wird das Wischelement in einem weiteren Schritt des erfindungsgemäßen Verfahrens, vorzugsweise durch leichtes mechanisches Aufdrücken seiner Wischfläche(n), mit dem den analytspezifischen Bindungspartner umfassenden zweiten Bereich des Testelements in Kontakt gebracht, wobei zumindest ein Teil der Probe von dem Wischelement auf das Testelement übertragen wird. Der Druck, mit dem das Wischelement auf das Testelement aufgedrückt wird, sollte dabei mindestens so groß sein, dass ein flächiger Kontakt zwischen der Oberfläche des Testelements und der Wischfläche/den Wischflächen des Wischelements besteht und insofern eine fluide Kommunikation zwischen beiden Elementen ermöglicht wird. Durch den direkten Kontakt zwischen Wischelement und dem zweiten Bereich des Testelements wird ein direkter und damit schneller Kontakt zwischen dem Analyten und dem analytspezifischen Bindungspartner realisiert, wodurch eine rasche Bildung des Komplexes aus Analyt und analytspezifischem Bindungspartner erfolgen kann und die Sensitivität und Spezifität des Nachweisverfahrens signifikant verbessert wird.

Um die Sensitivität und Spezifität des Verfahrens weiter zu verbessern, umfasst der zur Applikation der Probe ausgebildete zweite Bereich des Testelements in einer bevorzugten Variante zusätzlich mindestens ein Mittel, welches beispielsweise durch Blockierung bzw. Zerstörung unspezifischer Bindungsstellen in der Probe oder/und durch Veränderung der Probenkonsistenz eine chemische oder/und mechanische Aufbereitung der den Analyten enthaltenden Probe bewirkt. Auf diese Weise steht der Analyt optimal für eine Bindung an den analytspezifischen Bindungspartner (z.B. an einen Antikörper) zur Verfügung, womit die Zugänglichkeit des Analyten für den analytspezifischen Bindungspartner sowie der Transport über die einzelnen Bereiche des Testelements verbessert wird. Sofern ein chemisches Probenaufbereitungsmittel eingesetzt wird, so kann dieses beispielsweise auf dem Testelement imprägniert sein, wobei zur Imprägnierung vorzugsweise eine wässrige oder nicht-wässrige Lösung, welche das chemische Probenaufbereitungsmittel in einer Konzentration von etwa 0.01 bis etwa 5 Gew.-% enthält, eingesetzt wird. Besonders bevorzugt gelangen im Testelement mindestens ein chemisches Probenaufbereitungsmittel und mindestens ein mechanisches Probenaufbereitungsmittel parallel zur Anwendung.

Chemische Probenaufbereitungsmittel, welche im Rahmen des erfindungsgemäßen Verfahrens Anwendung finden können, umfassen insbesondere Säuren, Basen, Puffer, organische Lösungsmittel und Detergenzien, wobei Basen und Detergenzien als besonders bevorzugt gelten. Konkrete Beispiele für Säuren umfassen anorganische Säuren (z.B. Salzsäure) und organische Säuren (z.B. Essigsäure und Zitronensäure). Beispielhafte Basen umfassen insbesondere Alkali- und Erdalkalimetallhydroxide, wie etwa Natriumhydroxid und Calciumhydroxid, während als Puffer u.a. Calciumcarbonat, Tris, PBS, Phosphatpuffer, Boratpuffer, BICINE-Puffer und HEPES verwendet werden können. Beispiele für Detergenzien umfassen u.a. Octylglucosid, Cholamidopropansulfonat, Polidocanol, Polyalkylenglykolether (z.B. Brij^{®}, Synperonic^{®}) und Polysorbate (z.B. Tween^{®} 20, Tween^{®} 80). Beispielhafte organische Lösungsmittel umfassen insbesondere Dimethylsulfoxid, Ethanol, Glycerin, Isopropanol, Methanol, und Gemische hiervon.

Mechanische Probenaufbereitungsmittel, welche im Rahmen des erfindungsgemäßen Verfahrens Anwendung finden können, umfassen beispielsweise Gewebe oder/und Vliese, insbesondere Vliese, mittels welchen die Probe vor Inkubation des Analyten mit dem analytspezifischen Bindungspartner filtriert bzw. aufgetrennt wird, so dass speziell feste und viskose Probenbestandteile (z.B. feste und viskose Speichelbestandteile), die das Nachweisverfahren negativ beeinflussen können, zurückgehalten werden. Konkrete Beispiele für Vliese, welche eine mechanische Aufbereitung der Probe bewirken können, umfassen beispielsweise die kommerziell erhältlichen Produkte Ahlstrom 8964, Whatman Rapid 24Q und Freudenberg FS 2216, sind jedoch nicht auf diese beschränkt.

Um dem Benutzer das Inkontaktbringen von Testelement und Wischelement zu erleichtern, können in einer Ausführungsform der Erfindung ein oder mehrere Testelemente in einem Gehäuse untergebracht sein. Das Gehäuse weist hierbei vorzugsweise mindestens eine Aussparung auf, über welche das Wischelement mit dem zur Applikation der Probe ausgebildeten Bereich des jeweiligen Testelements in Kontakt gebracht werden kann. Sofern das Wischelement mehrere Wischflächen aufweist, so ist es als bevorzugt anzusehen, dass das Gehäuse lediglich eine einzige Aussparung zur Aufnahme des Wischelements enthält. Alternativ ist es indessen auch möglich, dass das Gehäuse eine der Anzahl an Wischflächen entsprechende Anzahl an Aussparungen enthält.

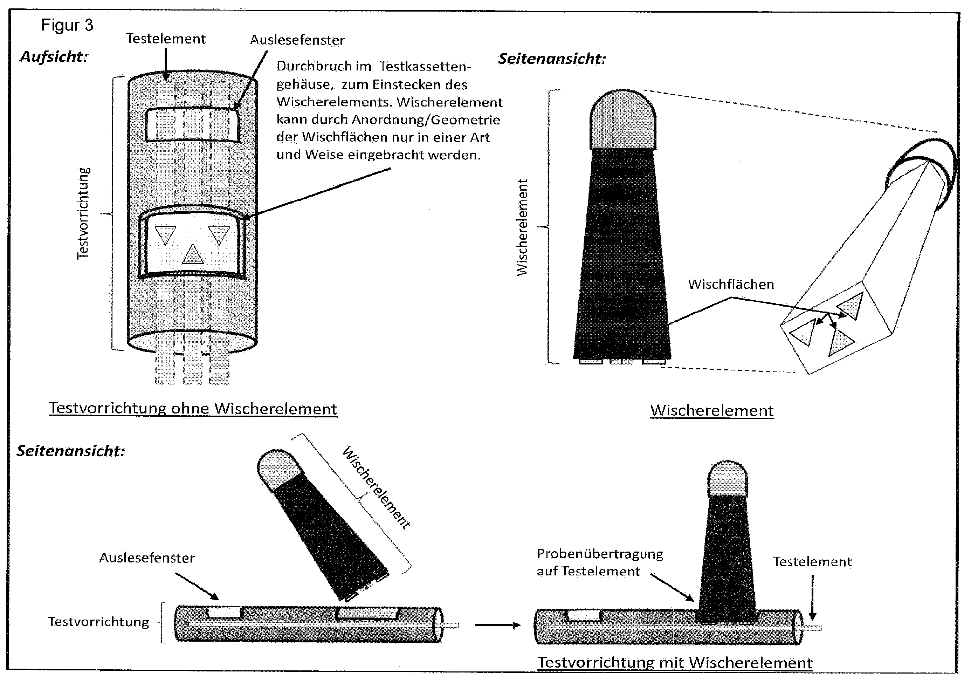

Die Aussparung(en) kann/können in beliebiger Form (zueinander) angeordnet sein und jede dem Fachmann geeignet erscheinende Dimension und Geometrie aufweisen, ist/sind jedoch üblicherweise an die Anordnung, die Dimension und die Geometrie der Wischfläche(n) des Wischelements angepasst. Hierdurch ist es beispielsweise möglich, eine vorab definierte Zusammenführung von Wischelement und Testelement zu erwirken, um auf diese Weise eine unsachgemäße Benutzung des Testsystems zu vermeiden (siehe Figur 3).

In einer weiteren Ausführungsform kann das Gehäuse ferner eine Haltevorrichtung umfassen, welche eine reversible Befestigung des Wischelements am Gehäuse gestattet, so dass letzteres beispielsweise zur Probennahme abgenommen und nach Probennahme wieder am Gehäuse angebracht werden kann. Hierbei kann durch Wahl einer geeigneten Position für die Haltevorrichtung das Wischelement derart auf dem Gehäuse platziert werden, dass ein intensiver Kontakt zwischen Wischelement bzw. dessen Wischfläche(n) und dem Testelement sichergestellt wird, was eine gute Übertragung des Analyten vom Wischelement auf das den analytspezifischen Bindungspartner umfassende Testelement gewährleistet.

Erfindungsgemäß werden das Testelement und das mit dem Analyten benetzte Wischelement zum Zwecke einer hohen Sensitivität und Spezifität der Analytenbestimmung für einen Zeitraum von mindestens 10 Sekunden miteinander in Kontakt gebracht, wobei der auf dem Testelement befindliche analytspezifische Bindungspartner mit dem zu bestimmenden Analyten inkubiert wird und gegebenenfalls zusätzlich eine chemische oder/und mechanische Aufbereitung der den Analyten enthaltenden Probe erfolgt. Auf diese Weise wird sichergestellt, dass der Analyt einerseits möglichst vollständig aus der Probenmatrix des Wischelements herausgelöst wird und andererseits nahezu quantitativ mit dem analytspezifischen Bindungspartner abreagieren kann. Als vorteilhaft hat sich in diesem Zusammenhang eine Inkubationszeit von etwa 10 Sekunden bis etwa 600 Sekunden, stärker bevorzugt von etwa 30 Sekunden bis etwa 300 Sekunden, am stärksten bevorzugt von etwa 60 Sekunden bis etwa 180 Sekunden, erwiesen. Durch eine derartige Verfahrensführung kann das für die Bestimmung des Analyten benötigte Probenvolumen üblicherweise auf weniger als 10 µl reduziert und die Sensitivität des Testsytems deutlich verbessert werden.

Im Anschluss an die Inkubation mit dem Analyten wird das Testelement mit einem Elutionsmittel in Kontakt gebracht. Zu diesem Zweck umfasst das Testelement vorzugsweise einen endständigen Bereich, welcher zur Aufnahme von Elutionsmittel ausgebildet ist und üblicherweise ein saugfähiges Material, wie beispielsweise Gewebe oder/und Vlies, umfasst. Nach Benetzung dieses Bereichs mit Elutionsmittel wandert das Elutionsmittel durch die verschiedenen Bereiche des Testelements, wobei Analyt, analytspezifischer Bindungspartner sowie deren Komplexe entsprechend mittransportiert werden. Hierbei kann vorteilhafterweise die Kapillarwirkung der einzelnen Komponenten des Testelements ausgenutzt werden, welche derart angeordnet bzw. miteinander verbunden sind, dass ein ununterbrochener Elutionsmittelfluss gewährleistet ist.

Als Elutionsmittel kann im Rahmen der vorliegenden Erfindung grundsätzlich jedes dem Fachmann als geeignet erscheinende Elutionsmittel verwendet werden. Vorzugsweise gelangen in dem hierin beschriebenen Verfahren allerdings Wasser und wässrige Pufferlösungen zur Anwendung, welche gegebenenfalls weitere Substanzen wie beispielsweise ein Kohlenhydrat, einen Zuckeralkohol, ein Detergenz oder/und ein organisches Lösungsmittel, wie jeweils oben beschrieben, in einer Konzentration von üblicherweise etwa 0.05 bis etwa 1.5 Gew.-% umfassen können. In einer bevorzugten Ausführungsform der Erfindung umfasst das Elutionsmittel einen Zuckeralkohol, wie er vorstehend definiert ist, oder/und ein Derivat hiervon, in welchem mindestens eine Hydroxygruppe des Zuckeralkohols gegen eine Mercaptogruppe ausgetauscht ist. Als besonders bevorzugt wird im Rahmen des erfindungsgemäßen Verfahrens ein vorzugsweise wässriges Elutionsmittel angesehen, welches 1,4-Dithioerythritol als Bestandteil umfasst, durch dessen Verwendung eine Erhöhung der Sensitivität oder/und Spezifität der Analytenbestimmung erzielt werden kann.

Sofern das Testelement in ein Gehäuse eingebracht ist, so bestehen in Abhängigkeit von der Ausgestaltung des Gehäuses verschiedene Möglichkeiten, eine Benetzung des Testelements mit Elutionsmittel zu erwirken. Ist das Testelement etwa vollständig in dem Gehäuse gelagert, so kann das Elutionsmittel beispielsweise durch Aufdrücken auf eine das Elutionsmittel enthaltende Ampulle, welche vorzugsweise innerhalb des Gehäuses gelagert ist, auf den zur Aufnahme von Elutionsmittel ausgebildeten ersten Bereich des Testelements aufgebracht werden. Ragt der zur Aufnahme von Elutionsmittel ausgebildete erste Bereich des Testelements indessen aus dem Gehäuse heraus, so besteht die Möglichkeit, den Bereich in das Elutionsmittel einzutauchen.

Nach Inkontaktbringen des Testelements mit dem Elutionsmittel wird die Bestimmung des Analyten in Gang gesetzt, welche vorzugsweise immunologisch erfolgt und beispielsweise ein kompetitives Testformat oder/und ein nicht-kompetitives Testformat (Sandwich-Testformat), insbesondere eine Kombination aus einem kompetitiven Testformat und einem nicht-kompetitiven Testformat, umfasst. Üblicherweise umfasst das erfindungsgemäße Nachweisverfahren zunächst die Bildung eines Komplexes aus Analytmolekülen und analytspezifischem, gegebenenfalls markiertem Bindungspartner, welcher mit Hilfe des Elutionsmittels, beispielsweise zusammen mit ungebundenem Bindungspartner oder/und weiteren in der Probe vorhandenen Substanzen, bis zum einem Bereich des Testelements weitertransportiert wird, der zur optischen Detektion des Analyten ausgebildet ist.

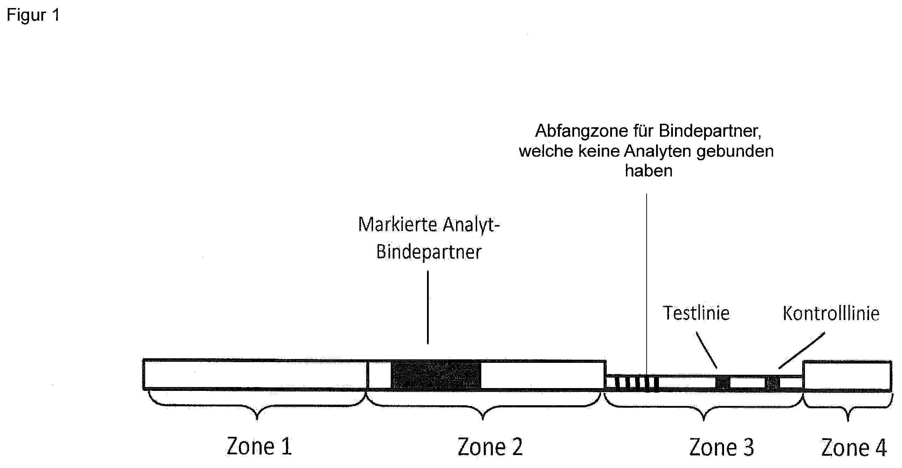

Der zur optischen Detektion des Analyten ausgebildete Bereich umfasst üblicherweise mehrere definierte Abschnitte, in welchen unterschiedliche Reagenzien immobilisiert sein können. In einer Ausführungsform umfasst dieser Bereich einen Abschnitt, welcher zur Bindung von ungebundenem analytspezifischem Bindungspartner ausgebildet ist, einen Abschnitt, welcher zur Bindung des Komplexes aus Analyt und analytspezifischem Bindungspartner ausgebildet ist, und gegebenenfalls einen Abschnitt, in welchem analytenunabhängig ein Kontrollsignal erzeugt wird (siehe Figur 1). Der zur optischen Detektion des Analyten ausgebildete Bereich kann dabei aus einem oder mehreren Materialien gebildet sein, welche dem Fachmann für die Zwecke der Erfindung geeignet erscheinen, wie beispielsweise Membranen aus Nylon^{®}, Nitrocellulose oder Polyvinylidenfluorid.

Der zur Bindung von ungebundenem analytspezifischem Bindungspartner vorgesehene Abschnitt kann beispielsweise immobilisierte Analytanaloga, insbesondere Polyhaptene, umfassen, welche den analytspezifischen, gegebenenfalls markierten Bindungspartner infolge Ausbildung eines Komplexes an einer definierten Position auf dem Testelement (Abfanglinie) abfangen und auf diese Weise die Erzeugung falsch-positiver Ergebnisse vermeiden. Der Komplex aus Analyt und analytspezifischem Bindungspartner wird an der Abfanglinie üblicherweise nicht immobilisiert, da der Analyt die hierzu erforderlichen Bindungsstellen am analytspezifischen Bindungspartner blockiert.

Der zur Bindung des Komplexes aus Analyt und analytspezifischem Bindungspartner ausgebildete Abschnitt umfasst vorzugsweise einen für den analytspezifischen Bindungspartner spezifischen, gegebenenfalls markierten Bindungspartner, welcher eine Immobilisierung des Komplexes aus Analyt und analytspezifischem Bindungspartner an einer vorbestimmten Position auf dem Testelement (Testlinie) bewirkt und auf diese Weise eine optische Bestimmung des Analyten ermöglicht. Die Immobilisierung des Komplexes erfolgt in der Regel über eine freie Bindungsstelle des analytspezifischen Bindungspartners, wobei eine Färbung der Testlinie mit einem positiven Nachweis des Analyten einhergeht.

Um das Signal an der Testlinie eindeutig ablesen zu können und Verwechslungen mit der Abfanglinie auszuschließen, kann die Abfanglinie gegebenenfalls in geeigneter Weise abgedeckt werden. Sofern das Testelement darüber hinaus einen Kontrollabschnitt umfasst, erscheint bei Durchführung des Verfahrens zusätzlich eine Kontrolllinie, die als Indikator für eine einwandfreie Funktionalität des Testelements fungiert. Überschüssiges Elutionsmittel, welches den zur optischen Detektion des Analyten ausgebildeten Bereich des Testelements verlässt, kann mit Hilfe eines flüssigkeitsabsorbierenden Materials in einem eigens hierfür ausgebildeten Bereich des Testelements aufgenommen werden.

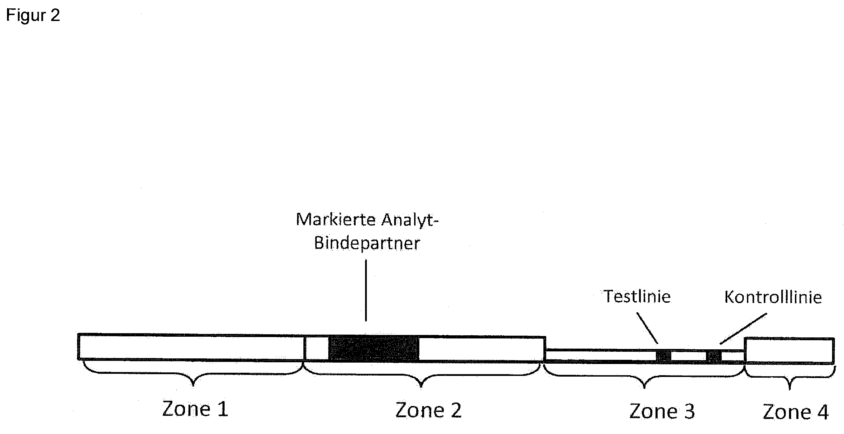

In einer anderen Ausführungsform umfasst der zur optischen Detektion des Analyten ausgebildete Bereich, wie er vorstehend beschrieben ist, keinen Abschnitt, welcher zur Bindung von ungebundenem analytspezifischem Bindungspartner ausgebildet ist (siehe Figur 2). In diesem Fall wird zur Erzeugung des Komplexes aus Analyt und analytspezifischem Bindungspartner vorzugsweise ein spezieller analytspezifischer Bindungspartner eingesetzt, wie er beispielsweise in EP 1 579 222 B1 beschrieben ist. Derartige Komplexe können in der Folge mittels eines komplexspezifischen Bindungspartners immobilisiert werden, was letztlich zu einer vereinfachten Detektion des Analyten führt, da die Erzeugung falsch-positiver Signale, welche u.a. durch nicht an der Abfanglinie gebundenen analytspezifischen Bindungspartner bewirkt werden können, vermieden wird.

Der zur Bindung des Komplexes aus Analyt und analytspezifischem Bindungspartner ausgebildete Abschnitt umfasst alsdann vorzugsweise einen komplexspezifischen Bindungspartner, welcher eine Immobilisierung des Komplexes aus Analyt und analytspezifischem Bindungspartner an einer vorbestimmten Position auf dem Testelement (Testlinie) bewirkt und auf diese Weise eine optische Bestimmung des Analyten ermöglicht. Die Immobilisierung des Komplexes aus Analyt und analytspezifischem Bindungspartner erfolgt in der Regel über eine freie Bindungsstelle des komplexspezifischen Bindungspartners, wobei eine Färbung der Testlinie mit einem positiven Nachweis des Analyten einhergeht.

Die im letzten Schritt des erfindungsgemäßen Verfahrens durchgeführte qualitative oder/und quantitative Bestimmung des Analyten kann auf beliebige Art und Weise erfolgen. Hierzu können grundsätzlich alle aus dem Stand der Technik bekannten Methoden zum Nachweis einer Komplexbildung eingesetzt werden, welche ein messbares Signal erzeugen, das manuell oder unter Verwendung geeigneter Mittel ausgewertet bzw. ausgelesen werden kann. Im Rahmen der vorliegenden Erfindung gelangen bevorzugt optische Nachweismethoden, insbesondere photometrische oder fluorimetrische Nachweismethoden, zum Einsatz. Besonders bevorzugt ist erfindungsgemäß ein optisch-visueller Nachweis des Analyten.

In einer besonders bevorzugten Ausführungsform werden mittels des erfindungsgemäßen Verfahrens mehrere unterschiedliche Analyten, insbesondere 2 bis 20 unterschiedliche Analyten, gleichzeitig bestimmt. In diesem Fall umfasst das Testelement in dem zur Applikation der Probe ausgebildeten zweiten Bereich üblicherweise eine der Anzahl an unterschiedlichen Analyten entsprechende Anzahl an unterschiedlichen analytspezifischen Bindungspartnern und, sofern das Testelement keinen Abschnitt zum Abfangen von ungebundenen analytspezifischen Bindungspartnern enthält, gegebenenfalls eine der Anzahl an unterschiedlichen Analyten entsprechende Anzahl an komplexspezifischen Bindungspartnern, wie sie vorstehend beschrieben sind. Auf diese Weise kann sichergestellt werden, dass die parallele Bestimmung unterschiedlicher Analyten auf dem Testelement im wesentlichen unabhängig voneinander erfolgt und keinerlei Interferenzen auftreten.

Das erfindungsgemäße Verfahren ermöglicht die Bestimmung eines oder mehrerer Analyten mit hoher Sensitivität und Spezifität. So ist es erfindungsgemäß bevorzugt, Analyten mit einer Spezifität von mindestens 95% oder/und einer Sensitivität von mindestens 90% zu bestimmen. Stärker bevorzugt erfolgt die Bestimmung mit einer Spezifität von mindestens 98% oder/und einer Sensitivität von mindestens 95%, so dass mittels des hierin beschriebenen Verfahrens Analyten bis hinab zu einer unteren Nachweisgrenze von etwa 1 ng/ml Probe nachgewiesen werden können.

Erfindungsgemäß ist es weiterhin bevorzugt, dass das im Rahmen des hierin beschriebenen Verfahrens verwendete Testelement oder/und Wischelement vor Kontakt mit der den Analyten enthaltenden Probe steril ist. Hierbei können das Testelement oder/und das Wischelement vor oder nach dem Sterilisieren in einen geeigneten Vorratsbehälter eingebracht werden, wobei ein entsprechendes Einbringen vor Durchführen der Sterilisation als bevorzugt anzusehen ist. Die Sterilisation selbst kann auf unterschiedliche Art und Weise erfolgen und umfasst beispielsweise chemische Sterilisation, Sterilisation durch Erhitzen und Sterilisation mittels ionisierender Strahlung. Das sterilisierte Testelement wird, gegebenenfalls zusammen mit dem Wischelement, im Anschluss an die Sterilisation vorzugsweise steril verpackt. Die sterile Verpackung ermöglicht es, sowohl Testelement als auch Wischelement bis zu einem späteren Gebrauch steril zu halten, ohne dass es einer erneuten Sterilisation bedarf. Besonders bevorzugt handelt es sich bei dem hierin beschriebenen Testelement bzw. Wischelement insofern um einen Einwegartikel, welcher nach Gebrauch nicht erneut verwendet wird.

Das erfindungsgemäße Verfahren kann zur Bestimmung einer beliebigen biologischen oder chemischen Substanz verwendet werden, welche beispielsweise unter Verwendung immunologischer Techniken nachweisbar ist. Vorzugsweise wird das hierin beschriebene Verfahren indessen zum Nachweis von Betäubungsmitteln, wie sie im deutschen Betäubungsmittelgesetz aufgelistet sind, verwendet. Konkrete Beispiele für derartige Betäubungsmittel umfassen u.a. Dissoziativa, Delirantia, Empathogene, Entaktogene, Hypnotika, Narkotika, Psychedelika, Sedativa, und Stimulantia, sind jedoch nicht auf diese beschränkt. In einer stärker bevorzugten Ausführungsform wird erfindungsgemäß mindestens ein Analyt ausgewählt aus der Gruppe bestehend aus Amphetaminen, Benzodiazepinen, Cannabinoiden, insbesondere Δ⁹-Tetrahydrocannabinol, Ketaminen, Methamphetaminen, Opiaten, insbesondere Morphin, Codein oder Dihydrocodein, natürlichen oder synthetischen Opioiden, insbesondere Heroin, und Tropan-Alkaloiden, insbesondere Kokain, bestimmt, wobei Δ⁹-Tetrahydrocannabinol und Kokain als Analyten besonders bevorzugt sind.

Der Analyt kann aus einer beliebigen Quelle stammen, wie beispielsweise von einer Oberfläche eines mit dem Analyten benetzten Gegenstandes oder aus einem Körperfluid, wie beispielsweise Vollblut, Plasma, Serum, Urin, Speichel oder Schweiß. Bevorzugt wird mittels des hierin beschriebenen Verfahrens das Vorhandensein oder/und die Menge eines Analyten in einer Probe aus Vollblut, Urin oder Speichel bestimmt. Die zur Durchführung des Verfahrens benötigte Probenmenge beträgt üblicherweise von etwa 0.01 µl bis etwa 100 µl, bevorzugt von etwa 0.05 µl bis etwa 20 µl, stärker bevorzugt von etwa 0.1 µl bis ≤ 10 µl, und am stärksten bevorzugt von etwa 0.5 µl bis etwa 5 µl.

In einem weiteren Aspekt betrifft die Erfindung ein Testelement zur Bestimmung eines Analyten, umfassend:
(i) einen ersten Bereich, welcher zur Aufnahme von Elutionsmittel ausgebildet ist,
(ii) einen zweiten Bereich, welcher zur Applikation einer den Analyten enthaltenden Probe ausgebildet ist und einen für den Analyten spezifischen Bindungspartner umfasst,
(iii) einen dritten Bereich, welcher zur optischen Detektion des Analyten ausgebildet ist,
(iv) einen vierten Bereich, welcher zur Aufnahme von überschüssigem Elutionsmittel ausgebildet ist,
(v) gegebenenfalls ein Gehäuse, und
(vi) ein Analyt-Übertragungsreagenz, welches mindestens eine analytunspezifische Substanz ausgewählt aus der Gruppe bestehend aus einem Protein, einem Proteingemisch, einem Kohlenhydrat und einem Zuckeralkohol enthält.

In noch einem weiteren Aspekt betrifft die Erfindung die Verwendung eines Wischelements in einem Verfahren wie oben beschrieben zur Aufnahme eines Analyten von einer Oberfläche und zur Übertragung des Analyten auf ein Testelement, wobei das Wischelement ein Analyt-Übertragungsreagenz umfasst, welches mindestens eine analytunspezifische Substanz ausgewählt aus der Gruppe bestehend aus einem Protein, einem Proteingemisch, einem Kohlenhydrat und einem Zuckeralkohol enthält. In noch einem weiteren Aspekt betrifft die Erfindung einen Kit zur Bestimmung eines Analyten, welcher vorzugsweise zur Durchführung des vorstehend beschriebenen Verfahrens verwendet wird und nachfolgende Bestandteile umfasst:
(a) ein Testelement, umfassend
   (i) einen ersten Bereich, welcher zur Aufnahme von Elutionsmittel ausgebildet ist,
   (ii) einen zweiten Bereich, welcher zur Applikation einer den Analyten (ii) einen zweiten Bereich, welcher zur Applikation einer den Analyten enthaltenden Probe ausgebildet ist und einen für den Analyten spezifischen Bindungspartner umfasst,
   (iii) einen dritten Bereich, welcher zur optischen Detektion des Analyten ausgebildet ist,
   (iv) einen vierten Bereich, welcher zur Aufnahme von überschüssigem Elutionsmittel ausgebildet ist, und
   (v) gegebenenfalls ein Gehäuse, und
(b) ein Wischelement, welches zur Aufnahme einer Probe des Analyten ausgebildet ist,
wobei das Testelement oder/und das Wischelement ein Analyt-Übertragungsreagenz umfasst, welches mindestens eine analytunspezifische Substanz ausgewählt aus der Gruppe bestehend aus einem Protein, einem Proteingemisch, einem Kohlenhydrat und einem Zuckeralkohol enthält.

Was bevorzugte Ausgestaltungen des erfindungsgemäßen Testelements, der erfindungsgemässen Verwendung des Wischelements und des in dem erfindungsgemäßen Kit enthaltenen Testelements bzw. Wischelements betrifft, so wird auf die Ausführungen in Zusammenhang mit der Beschreibung des erfindungsgemäßen Verfahrens verwiesen.

Die Erfindung soll durch die nachfolgenden Figuren und Beispiele näher erläutert werden:

### Beschreibung der Figuren

- **Figur 1:**: Querschnitt einer Ausführungsform eines Testelements zur Durchführung des Verfahrens gemäß der vorliegenden Erfindung. Das in Form eines Teststreifens dargestellte Testelement umfasst:
(a) einen ersten Bereich, der zur Aufnahme von Elutionsmittel ausgebildet ist (Zone 1),
(b) einen zweiten Bereich, der zur Applikation einer den Analyten enthaltenden Probe ausgebildet ist und einen analytspezifischen, markierten Bindungspartner sowie gegebenenfalls weitere Reagenzien umfasst (Zone 2),
(c) einen dritten Bereich, der zur optischen Detektion des Analyten ausgebildet ist und eine Abfanglinie, eine Testlinie und eine Kontrolllinie umfasst (Zone 3), und
(d) einen vierten Bereich, der zur Aufnahme von überschüssigem Elutionsmittel ausgebildet ist (Zone 4).
- **Figur 2:**: Querschnitt einer weiteren Ausführungsform eines Testelements zur Durchführung des Verfahrens gemäß der vorliegenden Erfindung. Das in Form eines Teststreifens dargestellte Testelement umfasst:
(a) einen ersten Bereich, der zur Aufnahme von Elutionsmittel ausgebildet ist (Zone 1),
(b) einen zweiten Bereich, der zur Applikation einer den Analyten enthaltenden Probe ausgebildet ist und einen analytspezifischen, markierten Bindungspartner sowie gegebenenfalls weitere Reagenzien umfasst (Zone 2),
(c) einen dritten Bereich, der zur optischen Detektion des Analyten ausgebildet ist und eine Testlinie sowie eine Kontrolllinie umfasst (Zone 3), und
(d) einen vierten Bereich, der zur Aufnahme von überschüssigem Elutionsmittel ausgebildet ist (Zone 4).
- **Figur 3:**: Darstellung einer Ausführungsform eines Wischelements und einer Testvorrichtung zur Durchführung des Verfahrens gemäß der vorliegenden Erfindung. Das Wischelement umfasst drei räumlich voneinander getrennte Wischflächen (jeweils in Dreiecksform) zur Aufnahme von mindestens 3 unterschiedlichen Analyten. Die Testvorrichtung umfasst drei räumlich voneinander getrennte Testelemente (jeweils in Form eines Teststreifens) sowie ein die Testelemente beinhaltendes Gehäuse, welches seinerseits eine Ausnehmung zum Ablesen der Testergebnisse sowie eine Ausnehmung zum Inkontaktbringen des Wischelements mit den Testelementen umfasst.

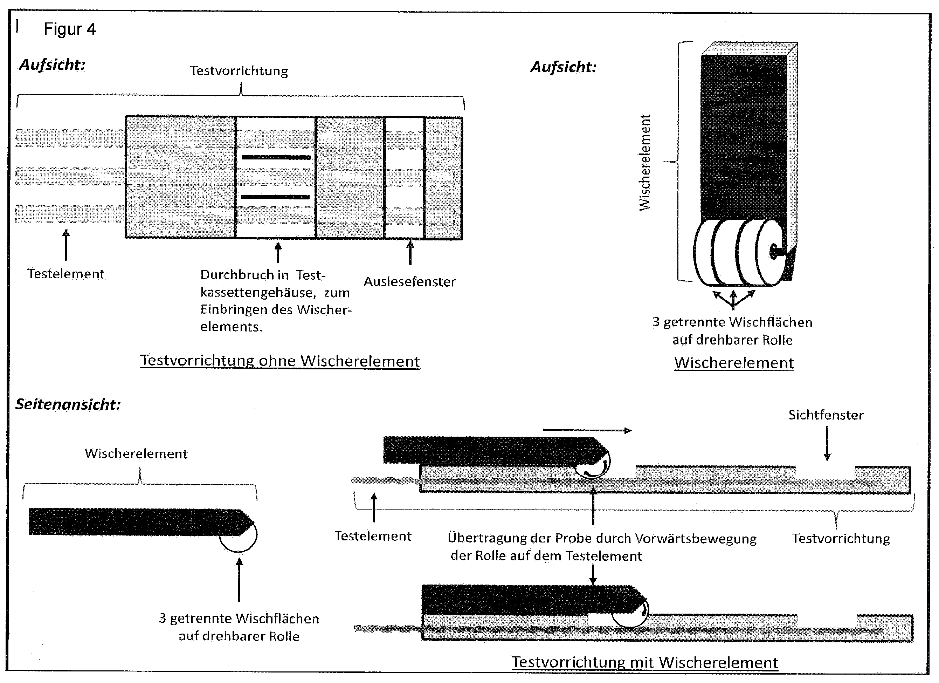

- **Figur 4:**: Darstellung einer weiteren Ausführungsform eines Wischelements und einer Testvorrichtung zur Durchführung des Verfahrens gemäß der vorliegenden Erfindung. Das Wischelement umfasst drei räumlich voneinander getrennte Wischflächen (jeweils in Rollenform) zur Aufnahme von mindestens 3 unterschiedlichen Analyten. Die Testvorrichtung umfasst drei räumlich voneinander getrennte Testelemente (jeweils in Form eines Teststreifens) sowie ein die Testelemente beinhaltendes Gehäuse, welches seinerseits eine Ausnehmung zum Ablesen der Testergebnisse sowie eine Ausnehmung zum Inkontaktbringen des Wischelements mit den Testelementen umfasst.

### Beispiele

### Beispiel 1: Herstellung von Δ⁹-THC-Immunogen

1.25 ml einer Stammlösung von Δ⁹-Tetrahydrocannabinolsäure A (1-Hydroxy-(-)-(6aR,10aR)-6,6,9-trimethyl-3-pentyl-6a,7,8,10a-tetrahydrobenzo[c]chromen-2-carbonsäure; Firma Lipomed) in N,N-Dimethylformamid (Konzentration: 100.8 mg/ml) wurden mit 8.75 ml N,N-Dimethylformamid auf ein Endvolumen von 10 ml verdünnt (Endkonzentration: 12.6 mg/ml).

5 ml dieser verdünnten Lösung wurden anschließend unter Rühren zu einer wässrigen Lösung von Rinderserumalbumin (BSA), welche vorab durch Lösen von 250 mg Rinderserumalbumin (Firma Sigma Aldrich) in einem Gemisch aus 25 ml destilliertem Wasser und 2.5 ml N,N-Dimethylformamid hergestellt worden war, hinzugefügt. Nach Zugabe von 6.3 ml destilliertem Wasser zu dem erhaltenen Reaktionsgemisch wurden aufeinander folgend die restlichen 5 ml der oben beschriebenen verdünnten Lösung von Δ⁹-Tetrahydrocannabinolsäure A in N,N-Dimethylformamid, weitere 6 ml destilliertes Wasser, sowie 0.2 ml einer Lösung von 1-Ethyl-3-(3-dimethylaminopropyl)carbodiimid-Hydrochlorid (Firma Sigma Aldrich) in N,N-Dimethylformamid (Konzentration 500 mg/ml) zugesetzt.

Das Becherglas mit der darin enthaltenen Lösung wurde in Aluminiumfolie eingewickelt und auf einem Magnetrührer für 24 h bei 5°C gerührt. Anschließend wurde die Lösung in einen Dialysebehälter überführt und über einen Zeitraum von 4 Tagen bei 5°C gegen eine 25% Lösung von N,N-Dimethylformamid in Wasser dialysiert. Das Dialysat wurde mittels Immunoelektrophorese getestet. Inkubation mit einem Ziege-anti-BSA-Antikörper (Firma Genetex) sowie eine Präzipitatbande zeigten, dass sich das Δ⁹-THC-Immunogen von der Bande des Rinderserumalbumins unterschied.

### Beispiel 2: Gewinnung von Antikörpern gegen Δ⁹-THC

20 Mäuse wurden mit dem in Beispiel 1 erhaltenen Δ⁹-THC-Immunogen in komplettem Freund-Adjuvans (Firma Sigma Aldrich) immunisiert, wobei die Dosis für die erste und jede weitere Immunisierung jeweils 75 µg Immunogen pro Tier betrug. Die Immunisierungen erfolgten über einen Zeitraum von 6 Monaten jeweils in einem Abstand von einem Monat.

Die aus den immunisierten Mäusen erhaltenen Seren wurden anschließend in einem Mikrotiterplatten-Assay auf die Anwesenheit von Antikörpern gegen Δ⁹-THC untersucht. Hierzu wurden mit Streptavidin beschichtete Mikrotiterplatten zunächst mit 10 ml einer Lösung von Δ⁹-Tetrahydrocannabinol-[N'-biotinylaminocaproyl-(3,6-dioxa-8-aminooctyl)-amid] in phosphatgepufferter Salzlösung, das vorab durch Umsetzung von Δ⁹-Tetrahydrocannabinolsuccinimidylester und N-(Biotinylaminocaproyl)-1,8-diamino-3,6-dioxaoctan (Firma Applichem) hergestellt worden war, für 12 h bei 4°C inkubiert.

Nach Waschen mit 20 ml einer 0.05% Lösung von Tween 20 in phosphatgepufferter Salzlösung wurden die Mikrotiterplatten jeweils für 1 h bei 20°C mit 10 ml der zu untersuchenden Seren inkubiert, und anschließend erneut mit 20 ml einer 0.05% Lösung von Tween 20 in phosphatgepufferter Salzlösung gewaschen. Zur Detektion wurde für 1 h bei 20°C mit 10 ml einer Lösung eines Konjugats aus Peroxidase und Kaninchen-anti-Maus-IgG (Firma Dako) inkubiert, mit 20 ml einer 0.05% Lösung von Tween 20 in phosphatgepufferter Salzlösung gewaschen, und mit Substrat versetzt. Seren mit einer guten Affinität gegenüber Δ⁹-THC wurden zur Gewinnnung von Antikörpern gegen Δ⁹-THC ausgewählt.

### Beispiel 3: Herstellung eines Gold-markierten Antikörpers gegen Δ⁹-THC

Goldsol mit einem durch Photonen-Korrelations-Spektroskopie bestimmten Partikeldurchmesser von 20 nm wurde in Anlehnung an den Stand der Technik (Frens, Nature (1973), 241, 20-22) hergestellt. Die Markierung der in Beispiel 2 gewonnenen, Δ⁹-THC-spezifischen Antikörper mit den Goldpartikeln erfolgte in Übereinstimmung mit bekannten Verfahren (Geoghegan et al., J. Immunol. Meth. (1980), 34, 11-31).

### Beispiel 4: Einsatz von Glucitol zur Sensitivitäts- und Spezifitätssteigerung

Ein aus Vlies gebildeter Bereich eines chromatographischen Teststreifens, welcher einen Δ⁹-THC spezifischen Antikörper gemäß Beispiel 3 enthielt, wurde mit drei unterschiedlich konzentrierten Lösungen von Glucitol (Firma Sigma) in destilliertem Wasser getränkt.

Anschließend wurde Poolspeichel (Speichelmix von fünf Personen) mit einer in Methanol verdünnten Lösung von Δ⁹-THC (Firma Cerilliant) auf eine Δ⁹-THC-Konzentration von 100 ng/ml eingestellt. Nach Auftragen von 10 µl dieses dotierten Poolspeichels auf die Wischfläche eines handelsüblichen Wischelements (Firma Securetec Detektionssysteme) wurde das Wischelement mit dem getränkten Bereich des chromatographischen Teststreifens in Kontakt gebracht und der Teststreifen für einen Zeitraum von 60 Sekunden bei Raumtemperatur inkubiert.

Nach Inkubation mit der Probe des Analyten wurde der Teststreifen für 15 Sekunden in Leitungswasser eingetaucht und der Chromatographieprozess hierdurch gestartet. Die vollständige Testentwicklung betrug 5 bis 10 Minuten. Die Ergebnisse der drei Bestimmungen sind in Tabelle 1 dargestellt:

**Tabelle 1**

| Glucitol-Konzentration (Gew.-% in der Tränkungslösung) | Testliniensignal für Δ⁹-THC |
|---|---|
| 2% | positiv |
| 1% | schwach positiv |
| 0% | negativ |

Wie aus Tabelle 1 hervorgeht, führt eine Tränkung des Vlieses mit steigenden Konzentrationen an Glucitol zu einer verbesserten Speichelübertragung vom Wischelement auf den chromatographischen Teststreifen. Gleichzeitig verbessert Glucitol das Herauslösen von Δ⁹-THC aus der Speichelprobe.

Beides zusammen ermöglicht einen verbesserten Kontakt zwischen Δ⁹-THC und dem in dem kontaktierten Bereich des Teststreifens vorgelegten markierten Antikörper, was eine deutlich spezifischere und sensitivere Bindung von Δ⁹-THC an den spezifischen Bindungspartner bewirkt und zu einer verstärkten Signalentwicklung an der Testlinie des chromatographischen Teststreifens führt.

### Beispiel 5: Einsatz von 1,4-Dithioerythritol im Elutionsmittel zur Sensitivitäts- und Spezifitätssteigerung

Poolspeichel (Speichelmix von fünf Personen) wurde mit einer in Methanol verdünnten Lösung von Δ⁹-THC (Firma Cerilliant) auf eine Δ⁹-THC-Konzentration von 80 ng/ml eingestellt. Nach Auftragen von 10 µl dieses dotierten Poolspeichels auf die Wischfläche eines handelsüblichen Wischelements (Firma Securetec Detektionssysteme) wurde das Wischelement mit einem aus Vlies gebildeten Bereich eines chromatographischen Teststreifens, welcher einen Δ⁹-THC spezifischen Antikörper gemäß Beispiel 3 enthielt, in Kontakt gebracht und der Teststreifen für einen Zeitraum von 60 Sekunden bei Raumtemperatur inkubiert.

Nach Inkubation mit der Probe des Analyten wurde der Teststreifen für 15 Sekunden in bidestilliertes Wasser, welches unter der Handelsbezeichnung Sputolysin^{®} (Firma VWR) kommerziell erhältliches 1,4-Dithioerythritol in vier unterschiedlichen Konzentrationen enthielt, eingetaucht und der Chromatographieprozess hierdurch gestartet. Die vollständige Testentwicklung betrug 5 bis 10 Minuten. Die Ergebnisse der vier Bestimmungen sind in Tabelle 2 dargestellt:

**Tabelle 2**

| 1,4-Dithioerythritol-Konzentration im Laufmittel (Gew.-%) | Testliniensignal für Δ⁹-THC |
|---|---|
| 5% | negativ |
| 1% | schwach positiv |
| 0.1% | positiv |
| 0% | schwach positiv |

Wie aus Tabelle 2 hervorgeht, führt eine Beimischung von 1,4-Dithioerythritol zum Elutionsmittel in einem Konzentrationsbereich von etwa 0.01 bis etwa 1 Gew.-% zu einer verstärkten Signalentwicklung an der Testlinie des chromatographischen Teststreifens.

### Beispiel 6: Einsatz von Natriumhydroxid zur Sensitivitäts- und Spezifitätssteigerung

Ein aus Vlies gebildeter Bereich eines chromatographischen Teststreifens wurde mit einer Lösung von 2.0 Gew.-% Rinderserumalbumin (Firma Sigma Aldrich), 0.5 Gew.-% Brij^{®} (Firma Sigma Aldrich) und 0.03 Gew.-% Natriumhydroxid in destilliertem Wasser getränkt (Vlies A). Um den Effekt von Natriumhydroxid zum Zwecke einer chemischen Speichelaufbereitung zu evaluieren, wurde parallel ein aus Vlies gebildeter Bereich eines zweiten chromatographischen Teststreifens mit einer Lösung von 2.0 Gew.-% Rinderserumalbumin und 0.5 Gew.-% Brij^{®} in destilliertem Wasser getränkt (Vlies B).

Anschließend wurde Poolspeichel (Speichelmix von fünf Personen) mit einer in Methanol verdünnten Lösung von Benzodiazepin (Firma Cerilliant) auf eine Benzodiazepin-Konzentration von 20 ng/ml, 30 ng/ml, 50 ng/ml bzw. 100 ng/ml eingestellt. Nach Auftragen von 10 µl dieses dotierten Poolspeichels auf die Wischfläche eines handelsüblichen Wischelements (Firma Securetec Detektionssysteme) wurde das Wischelement mit dem getränkten Bereich der beiden chromatographischen Teststreifen, welcher jeweils keinen Benzodiazepin-spezifischen Antikörper enthielt, in Kontakt gebracht und die Teststreifen für einen Zeitraum von 30 Sekunden bei Raumtemperatur inkubiert.

Nach Inkubation mit der Probe des Analyten wurde der Teststreifen in bidestilliertes Wasser eingetaucht und der Chromatographieprozess hierdurch gestartet. Die vollständige Testentwicklung betrug 5 bis 10 Minuten. Die Ergebnisse der Bestimmungen sind in Tabelle 3 dargestellt:

**Tabelle 3**

| Benzodiazepin-Konzentration | Testliniensignal für Benzodiazepin | |
|---|---|---|
| | Vlies A | Vlies B |
| 20 ng/ml | schwach positiv | negativ |
| 30 ng/ml | positiv | negativ |
| 50 ng/ml | positiv | negativ |
| 100 ng/ml | positiv | positiv |

Wie aus Tabelle 3 hervorgeht, führt eine Tränkung des Vlieses mit geringen Mengen an Natriumhydroxid zu einer verstärkten Signalentwicklung an der Testlinie des chromatographischen Teststreifens und damit zu einer Verbesserung des Benzodiazepin-Nachweises.

### Beispiel 7: Einsatz von Vliesen zur Sensitivitäts- und Spezifitätssteigerung

Um den Effekt unterschiedlicher Vliese für die Zwecke einer mechanischen Speichelaufbereitung zu evaluieren, wurde chromatographische Teststreifen bereitgestellt, welche einen aus Sontara^{®} 8423 (Firma DuPont), Ahlstrom 8964 (Firma Ahlstrom), Rapid 24Q (Firma Whatman) bzw. FS 2216 (Firma Freudenberg) gebildeten Bereich zur Aufnahme einer zu untersuchenden Probe umfassten.

Poolspeichel (Speichelmix von fünf Personen) wurde mit einer in Methanol verdünnten Lösung von Kokain (Firma Cerilliant) auf eine Kokain-Konzentration von 5 ng/ml, 15 ng/ml bzw. 50 ng/ml eingestellt. Nach Auftragen von 10 µl dieses dotierten Poolspeichels auf die Wischfläche eines handelsüblichen Wischelements (Firma Securetec Detektionssysteme) wurde das Wischelement jeweils mit dem aus Vlies gebildeten Bereich der chromatographischen Teststreifen, welcher jeweils keinen Kokainspezifischen Antikörper enthielt, in Kontakt gebracht und die Teststreifen für einen Zeitraum von 60 Sekunden bei Raumtemperatur inkubiert.

Nach Inkubation mit der Probe des Analyten wurde der Teststreifen in bidestilliertes Wasser eingetaucht und der Chromatographieprozess hierdurch gestartet. Die vollständige Testentwicklung betrug 5 bis 10 Minuten. Die Ergebnisse der Bestimmungen sind in Tabelle 4 dargestellt:

**Tabelle 4**

| Kokain-Konzentration | Testliniensignal für Kokain | | | |
|---|---|---|---|---|
| | Sontara^{®} 8423 | Ahlstrom 8964 | Rapid 24Q | FS 2216 |
| 5 ng/ml | negativ | schwach positiv | schwach positiv | schwach positiv |
| 15 ng/ml | schwach positiv | positiv | positiv | positiv |
| 50 ng/ml | schwach positiv | positiv | positiv | positiv |

Wie aus Tabelle 4 hervorgeht, führt die Verwendung des Ahlstrom 8964-Vlieses, des Whatman Rapid 24Q-Vlieses sowie des Freudenberg FS 2216-Vlieses bereits bei einer Kokain-Konzentration von 5 ng/ml zu einer verstärkten Signalentwicklung an der Testlinie des chromatographischen Teststreifens und damit zu einer Verbesserung des Kokain-Nachweises. Dies ist im wesentlichen darauf zurückzuführen, dass Ahlstrom^{®}8964, Whatman Rapid 24Q und Freudenberg FS 2216 die festen bzw. viskosen Speichelbestandteile besonders gut mechanisch zurückhalten und insofern ein verbessertes Chromatographieverhalten zeigen.

### Beispiel 8: Einfluss der Inkubation von Analyt und analytspezifischem Bindungspartner auf die Sensitivität

Im Rahmen einer klinischen Studie wurde Methadon-konsumierenden Probanden, welche maximal 8 Stunden vor der Beprobung zusätzlich Δ⁹-THC zu sich genommen hatten und einen sehr trockenen Mund ohne ersichtlich feuchten Speichel aufwiesen, mittels eines handelsüblichen Wischelements (Firma Securetec Detektionssysteme) eine Speichelprobe entnommen. Als Negativkontrolle dienten Probanden, welche vor der Beprobung kein Δ⁹-THC konsumiert hatten. Zur Bestimmung des jeweils aufgenommenen Volumens an Speichel wurden die Wischelemente vor und nach der Beprobung gewogen.

Um den Effekt einer direkten Inkubation von Analyt und analytspezifischem Bindungspartner auf die Sensitivität des Nachweisverfahrens zu evaluieren, wurden die Wischelemente in einer ersten Versuchsreihe jeweils mit einem aus Vlies gebildeten Bereich eines chromatographischen Teststreifens, wie er in EP 0 699 906 A2 und EP 0 811 842 A2 offenbart ist, in Kontakt gebracht. Da die in vorstehenden Druckschriften offenbarten Teststreifen in dem zur Aufnahme der Probe ausgebildeten Bereich keinen analytspezifischen Bindungspartner enthalten, können Analyt und analytspezifischer Bindungspartner in beiden Fällen erst nach dem Start der Chromatographie miteinander in Kontakt treten.

In einer zweiten Versuchsreihe wurden die Wischelemente jeweils mit einem aus Vlies gebildeten Bereich eines erfindungsgemäßen chromatographischen Teststreifens, welcher in dem zur Aufnahme der Probe ausgebildeten Bereich einen Δ⁹-THC spezifischen Antikörper gemäß Beispiel 3 enthielt, in Kontakt gebracht und die Teststreifen für einen Zeitraum von 60 Sekunden bei Raumtemperatur inkubiert. Anschließend wurden sämtliche Teststreifen jeweils in bidestilliertes Wasser eingetaucht und der Chromatographieprozess gestartet. Die vollständige Testentwicklung betrug 5 bis 10 Minuten. Die Ergebnisse sind in Tabelle 5 dargestellt:

**Tabelle 5**

| Probandennummer | Speicheivolumen [µl] | Testliniensignal mit Inkubation | Testliniensignal ohne Inkubation |
|---|---|---|---|
| 1 | 2.1 | positiv | positiv |
| 2 | 1.4 | positiv | negativ |
| 3 | 1.0 | schwach positiv | negativ |
| 4 | 1.5 | positiv | schwach positiv |
| 5 | 0.8 | positiv | negativ |
| 6 | 1.1 | negativ | negativ |
| 7 | 1.2 | schwach positiv | negativ |
| 8 | 1.4 | positiv | negativ |
| 9 | 1.5 | schwach positiv | negativ |
| 10 | 2.0 | positiv | positiv |
| 11 (Negativkontrolle) | 1.9 | negativ | negativ |
| 12 (Negativkontrolle) | 1.4 | negativ | negativ |
| 13 (Negativkontrolle) | 1.2 | negativ | negativ |

Wie aus Tabelle 5 hervorgeht, kann durch eine 60-sekündige Inkubation von Analyt und analytspezifischem Bindungspartner vor dem Start des Chromatographieprozesses selbst bei Probanden mit sehr trockenem Mund (Mittelwert des Probenvolumens bei den Positivkontrollen: 1.4 µl; Standardabweichung: 0.4 µl) eine 3-fach höhere Anzahl an Δ⁹-THC-Konsumenten erkannt werden als im Falle herkömmlicher Testsysteme, welche keine Inkubation von Analyt und analytspezifischem Bindungspartner vor dem Start der Chromatographie umfassen.

### Beispiel 9: Einfluss der Inkubationszeit von Analyt und analytspezifischem Bindungspartner auf die Sensitivität

Poolspeichel (Speichelmix von fünf Personen) wurde mit einer in Methanol verdünnten Lösung von Δ⁹-THC (Firma Cerilliant) zunächst auf eine Δ⁹-THC-Konzentration von 200 ng/ml eingestellt. Im Anschluss wurden 8 µl dieses dotierten Poolspeichels mit einem handelsüblichen Wischelement (Firma Securetec Detektionssysteme) aufgenommen.

Im Anschluss wurde das auf diese Weise erhaltene Wischelement in zwei Versuchsreihen für einen Zeitraum von 10, 20, 40, 60, 100 bzw. 180 Sekunden mit einem aus Vlies gebildeten Bereich eines chromatographischen Teststreifens in Kontakt gebracht, wobei im Rahmen der ersten Versuchsreihe jeweils chromatographische Teststreifen verwendet wurden, welche in dem zur Aufnahme der Probe ausgebildeten Bereich einen Δ⁹-THC spezifischen Antikörper gemäß Beispiel 3 enthielten.

Parallel hierzu wurde eine zweite Versuchsreihe unter Verwendung chromatographischer Teststreifen durchgeführt, welche den Δ⁹-THC spezifischen Antikörper gemäß Beispiel 3 nicht in dem zur Aufnahme der Probe ausgebildeten Bereich, sondern in einem hiervon räumlich abgetrennten Bereich enthielten, so dass ein Kontakt zwischen dem Analyten und dem analytspezifischen Bindungspartner erst im Verlauf der Chromatographie realisiert wurde.

Anschließend wurden sämtliche Teststreifen jeweils in bidestilliertes Wasser eingetaucht und der Chromatographieprozess gestartet. Die vollständige Testentwicklung betrug 5 bis 10 Minuten. Die Ergebnisse sind in Tabelle 6 dargestellt:

**Tabelle 6**

| Inkubationszeit [sec] | Testliniensignal mit Δ⁹-THC-spezifischem Antikörper im Aufnahmebereich | Testliniensignal ohne Δ⁹-THC-spezifischen Antikörper im Aufnahmebereich |
|---|---|---|
| 10 | ++ | + |
| 20 | ++ | + |
| 40 | ++ | (+) |
| 60 | ++ | (+) |
| 100 | ++ | ((+)) |
| 180 | ++ | ((+)) |

| | | |
|---|---|---|
| ++ stark positives Signal + positives Signal (+) schwach positives Signal ((+)) grenzwertiges positives Signal - Übergang zu negativem Signal | | |

Wie aus Tabelle 6 hervorgeht, kann im Falle von chromatographischen Teststreifen, welche den Δ⁹-THC spezifischen Antikörper in dem zur Aufnahme der Probe ausgebildeten Bereich enthalten, bereits durch eine 10-sekündige Inkubation von Analyt und analytspezifischem Bindungspartner vor dem Start der Chromatographie eine starke Signalentwicklung an der Testlinie des chromatographischen Teststreifens erzielt werden. Gleichzeitig ist ersichtlich, dass sich die Stärke des Testliniensignals mit zunehmender Inkubationszeit nicht verändert.

Letzteres ist zum einen darauf zurückzuführen, dass der Analyt unmittelbar nach Inkontaktbringen von Wischelement und chromatographischem Teststreifen einen Komplex mit dem analytspezifischen Bindungspartner bildet und somit nicht oder nur zu einem kleinen Teil unspezifisch an das Vliesmaterial des Teststreifens bindet. Andererseits zeigt sich mit zunehmender Inkubationszeit auch keine Verstärkung des Testliniensignals, da die relativ hohe Analytkonzentration von 200 ng/ml den positiven Effekt einer verlängerten Inkubationszeit verdeckt.

Bei Wiederholung obiger Versuchsreihen unter Verwendung einer Δ⁹-THC-Konzentration von 20 ng/ml werden die in Tabelle 7 dargestellten Ergebnisse erhalten:

**Tabelle 7**

| Inkubationszeit [sec] | Testliniensignal mit Δ⁹-THC-spezifischem Antikörper im Aufnahmebereich | Testliniensignal ohne Δ⁹-THC-spezifischen Antikörper im Aufnahmebereich |
|---|---|---|
| 10 | ((+)) | - |
| 20 | (+) | - |
| 40 | (+) | - |
| 60 | + | - |
| 100 | ++ | - |
| 180 | ++ | - |

| | | |
|---|---|---|
| ++ stark positives Signal + positives Signal (+) schwach positives Signal ((+)) grenzwertiges positives Signal - Übergang zu negativem Signal - negatives Signal | | |

Wie aus Tabelle 7 hervorgeht, kann Δ⁹-THC im Falle chromatographischer Teststreifen, welche den Δ⁹-THC spezifischen Antikörper in dem zur Aufnahme der Probe ausgebildeten Bereich enthalten und damit eine direkte Inkubation von Analyt und analytspezifischem Bindungspartner ermöglichen, auch in einer Konzentration von lediglich 20 ng/ml bereits nach geringer Inkubationszeit detektiert werden.

Mit Zunahme der Inkubationszeit nimmt die Intensität des Testliniensignals zu, wobei ab einer Inkubationszeit von 100 Sekunden das maximale Testliniensignal erreicht wird. Gleichzeitig ist aus Tabelle 7 ersichtlich, dass im Falle der Testvariante ohne Δ⁹-THC spezifischen Antikörper im Probenaufnahmebereich des chromatographischen Teststreifens der Nachweis von Δ⁹-THC in einer Konzentration von 20 ng/ml nicht möglich ist.

## Patentansprüche

1. Verfahren zur Bestimmung eines Analyten, umfassend die Schritte:
(a) Bereitstellen eines Testelements, umfassend
(i) einen ersten Bereich, welcher zur Aufnahme von Elutionsmittel ausgebildet ist,
(ii) einen zweiten Bereich, welcher zur Applikation einer den Analyten enthaltenden Probe ausgebildet ist und einen für den Analyten spezifischen Bindungspartner umfasst,
(iii) einen dritten Bereich, welcher zur optischen Detektion des Analyten ausgebildet ist,
(iv) einen vierten Bereich, welcher zur Aufnahme von überschüssigem Elutionsmittel ausgebildet ist, und
(v) gegebenenfalls ein Gehäuse,
(b) Aufnehmen einer den Analyten enthaltenden Probe von einer Probenoberfläche mit einem Wischelement,
(c) Inkontaktbringen des Wischelements mit dem zweiten Bereich des Testelements für einen Zeitraum von mindestens 10 Sekunden, wobei zumindest ein Teil der Probe von dem Wischelement auf das Testelement übertragen wird und eine Inkubation des Analyten mit dem analytspezifischen Bindungspartner erfolgt,
(d) Inkontaktbringen des ersten Bereichs des inkubierten Testelements mit einem Elutionsmittel, und
(e) Bestimmen des Vorhandenseins oder/und der Menge des Analyten, wobei das Testelement oder/und das Wischelement ein Analyt-Übertragungsreagenz umfasst, welches mindestens eine analytunspezifische Substanz ausgewählt aus der Gruppe bestehend aus einem Protein, einem Proteingemisch, einem Kohlenhydrat und einem Zuckeralkohol enthält.

2. Verfahren nach Anspruch 1,
**dadurch gekennzeichnet,**
**dass** als Testelement ein chromatographischer Teststreifen verwendet wird.

3. Verfahren nach Anspruch 1 oder 2,
**dadurch gekennzeichnet,**
**dass** als analytspezifischer Bindungspartner ein Antikörper oder ein funktionelles Antikörperfragment verwendet wird.

4. Verfahren nach einem der Ansprüche 1 bis 3,
**dadurch gekennzeichnet,**
**dass** ein Wischelement mit einer oder mit mehreren Wischflächen verwendet wird, wobei das Wischelement und das Testelement insbesondere für einen Zeitraum von etwa 10 Sekunden bis etwa 600 Sekunden, oder von etwa 60 Sekunden bis etwa 180 Sekunden, miteinander in Kontakt gebracht werden.

5. Verfahren nach einem der Ansprüche 1 bis 4,
**dadurch gekennzeichnet,**
**dass** das Testelement ein Analyt-Übertragungsreagenz umfasst, welches mindestens eine analytunspezifische Substanz ausgewählt aus der Gruppe bestehend aus einem Kohlenhydrat und einem Zuckeralkohol enthält, und/oder das Wischelement ein Analyt-Übertragungsreagenz umfasst, welches mindestens eine analytunspezifische Substanz ausgewählt aus der Gruppe bestehend aus einem Protein und einem Proteingemisch enthält.

6. Verfahren nach einem der Ansprüche 1 bis 5,
**dadurch gekennzeichnet,**
**dass** der zweite Bereich des Testelements weiterhin mindestens ein Mittel zur chemischen oder/und mechanischen Aufbereitung der den Analyten enthaltenden Probe umfasst, wobei das chemische Probenaufbereitungsmittel insbesondere eine Substanz ausgewählt aus der Gruppe bestehend aus einer Säure, einer Base, einem Puffer, einem organischen Lösungsmittel und einem Detergenz umfasst, und wobei das mechanische Probenaufbereitungsmittel insbesondere ein Gewebe oder/und ein Vlies umfasst.

7. Verfahren nach einem der Ansprüche 1 bis 6,
**dadurch gekennzeichnet,**
**dass** es eine Kombination aus einem kompetitiven Testformat und einem nicht-kompetitiven Testformat umfasst.

8. Verfahren nach einem der Ansprüche 1 bis 7,
**dadurch gekennzeichnet,**
**dass** der Analyt mit einer Spezifität von mindestens 95% oder/und mit einer Sensitivität von mindestens 90% bestimmt wird.

9. Verfahren nach einem der Ansprüche 1 bis 8,
**dadurch gekennzeichnet,**
**dass** als Analyt ein Betäubungsmittel, insbesondere eine Substanz ausgewählt aus der Gruppe bestehend aus Dissoziativa, Delirantia, Empathogenen, Entaktogenen, Hypnotika, Narkotika, Psychedelika, Sedativa und Stimulantia, bestimmt wird.

10. Verfahren nach einem der Ansprüche 1 bis 9,
**dadurch gekennzeichnet,**
**dass** mehrere Analyten, insbesondere 2 bis 20 unterschiedliche Analyten, gleichzeitig bestimmt werden.

11. Verfahren nach einem der Ansprüche 1 bis 10,
**dadurch gekennzeichnet,**
**dass** als Probe ein Körperfluid, insbesondere Blut, Urin oder Speichel, verwendet wird.

12. Verfahren nach einem der Ansprüche 1 bis 11,
**dadurch gekennzeichnet,**
**dass** eine Probe mit einem Volumen von etwa 0.05 µl bis etwa 20 µl, insbesondere mit einem Volumen von etwa 0.1 µl bis etwa 5 µl, verwendet wird.

13. Testelement zur Bestimmung eines Analyten, umfassend:
(i) einen ersten Bereich, welcher zur Aufnahme von Elutionsmittel ausgebildet ist,
(ii) einen zweiten Bereich, welcher zur Applikation einer den Analyten enthaltenden Probe ausgebildet ist und einen für den Analyten spezifischen Bindungspartner umfasst,
(iii) einen dritten Bereich, welcher zur optischen Detektion des Analyten ausgebildet ist,
(iv) einen vierten Bereich, welcher zur Aufnahme von überschüssigem Elutionsmittel ausgebildet ist,
(v) gegebenenfalls ein Gehäuse, und
(vi) ein Analyt-Übertragungsreagenz, welches mindestens eine analytunspezifische Substanz ausgewählt aus der Gruppe bestehend aus einem Protein, einem Proteingemisch, einem Kohlenhydrat und einem Zuckeralkohol enthält.

14. Verwendung eines Wischelements in einem Verfahren nach einem der Ansprüche 1 bis 12 zur Aufnahme eines Analyten von einer Oberfläche und zur Übertragung des Analyten auf ein Testelement, wobei das Wischelement ein Analyt-Übertragungsreagenz umfasst, welches mindestens eine analytunspezifische Substanz ausgewählt aus der Gruppe bestehend aus einem Protein, einem Proteingemisch, einem Kohlenhydrat und einem Zuckeralkohol enthält.

15. Kit zur Bestimmung eines Analyten, umfassend:
(a) ein Testelement, umfassend
(i) einen ersten Bereich, welcher zur Aufnahme von Elutionsmittel ausgebildet ist,
(ii) einen zweiten Bereich, welcher zur Applikation einer den Analyten enthaltenden Probe ausgebildet ist und einen für den Analyten spezifischen Bindungspartner umfasst,
(iii) einen dritten Bereich, welcher zur optischen Detektion des Analyten ausgebildet ist,
(iv) einen vierten Bereich, welcher zur Aufnahme von überschüssigem Elutionsmittel ausgebildet ist, und
(v) gegebenenfalls ein Gehäuse, und
(b) ein Wischelement, welches zur Aufnahme einer Probe des Analyten ausgebildet ist,
wobei das Testelement oder/und das Wischelement ein Analyt-Übertragungsreagenz umfasst, welches mindestens eine analytunspezifische Substanz ausgewählt aus der Gruppe bestehend aus einem Protein, einem Proteingemisch, einem Kohlenhydrat und einem Zuckeralkohol enthält.

## Claims

1. Method for determining an analyte, comprising the steps of:
(a) providing a test element, comprising
(i) a first region which is adapted to receive eluent,
(ii) a second region which is adapted for applying a sample containing the analyte, and comprises an analyte specific binding partner,
(iii) a third region which is adapted for the optical detection of the analyte,
(iv) a fourth region which is adapted for absorbing excess ofeluent, and
(v) optionally a housing,
(b) receiving a sample containing the analyte of a sample surface with a wiping element,
(c) contacting the wiping element with the second region of the test element for a period of at least 10 seconds, at least a portion of the sample from the wiping element being transferred to the test element, and an incubation of the analyte with the analyte specific binding partner occurs,
(d) contacting the first region of the incubated test element with an eluent, and
(e) determining the presence and/or amount of the analyte,
wherein the test element and/or the wiping element comprises an analyte-transfer agent containing at least one analyte specific substance selected from the group consisting of a protein, a protein mixture, a carbohydrate and a sugar alcohol.

2. Method of claim 1, **characterized in that** a chromatographic test strip is used as test element.

3. Method of claim 1 or 2, **characterized in that** an antibody or a functional antibody fragment is used as analyte specific binding partner.

4. Method according to one of claims 1 to 3, **characterized in that** a wiping element is used with one or more wiping surfaces, wherein the wiper element and the test element are brought into contact with each other for a period of about 10 seconds to about 600 seconds, in particular of about 60 seconds to about 180 seconds.

5. Method according to one of claims 1 to 4, **characterized in that** the test element comprises an analyte transfer agent containing at least one analyte specific substance selected from the group consisting of a carbohydrate and a sugar alcohol, and/or that the wiper element comprises an analyte transfer agent containing at least one analyte specific substance selected from the group consisting of a protein and a protein mixture.

6. Method according to one of claims 1 to 5, **characterized in that** the second region of the test element further comprising at least one agent for the chemical and/or mechanical preparation of the sample containing the analyte, wherein the chemical sample preparation agent comprises in particular a substance selected from the group consisting of an acid, a base, a buffer, an organic solvent and a detergent, and wherein the mechanical sample preparation agent comprises a fabric and/or a nonwoven.

7. Method according to one of claims 1 to 6, **characterized in that** it comprises a combination of a competitive test format and a non-competitive test format.

8. Method according to one of claims 1 to 7, **characterized in that** the analyte is determined with a specificity of at least 95% and/or with a sensitivity of at least 90%.

9. Method according to one of claims 1 to 8, **characterized in that** a narcotic drug, in particular a substance selected from the group consisting of dissociatives, deliriants, empathogens, entactogens, hypnotics, narcotics, psychedelics, sedatives and stimulants is determined as analyte.

10. Method according to one of claims 1 to 9, **characterized in that** many analytes, in particular 2 to 20 different analytes are determined simultaneously.

11. Method according to one of claims 1 to 10, **characterized in that** a body fluid, in particular blood, urine or saliva, is used as a sample.

12. Method according to one of claims 1 to 11, **characterized in that** a sample having a volume of about 0.05 µl to about 20 µl, and in particular having a volume of about 0.1 µl to about 5 µl is used.

13. Test element for determining an analyte, comprising:
(i) a first region which is adapted to receive eluent,
(ii) a second region which is adapted for applying a sample containing the analyte, and comprises an analyte specific binding partner,
(iii) a third region which is adapted for the optical detection of the analyte,
(iv) a fourth region which is adapted for absorbing excess of eluent, and
(v) optionally a housing,
(vi) an analyte transfer reagent containing at least one analyte specific substance selected from the group consisting of a protein, a protein mixture, a carbohydrate and a sugar alcohol.

14. Use of a wiping element in a method according to one of claims 1 to 12 for receiving an analyte from a surface and for transfering the analyte on the test element, wherein the wiping element comprises an analyte transfer reagent which contains at least one analyte specific substance selected from the group consisting of a protein, a protein mixture, a carbohydrate and a sugar alcohol.

15. Kit for determining an analyte, comprising:
(a) a test element, comprising
(i) a first region which is adapted to receive eluent,
(ii) a second region which is adapted for applying a sample containing the analyte, and comprises an analyte specific binding partner,
(iii) a third region which is adapted for the optical detection of the analyte,
(iv) a fourth region which is adapted for absorbing excess of eluent, and
(v) optionally a housing,
(b) a wiper element for receiving a sample of the analyte
wherein the test element and/or the wiper element comprises an analyte-transfer agent, containing at least one analyte transfer substance selected from the group consisting of a protein, a protein mixture, a carbohydrate and a sugar alcohol.

## Revendications

1. Procédé de détermination d'un analyte, comprenant les étapes de:
(a) fournir un élément de test, comprenant:
(i) un premier domaine qui est adapté pour l'absorption d'un agent d'élution,
(ii) un deuxième domaine qui est adapté pour l'application d'un échantillon contenant un analyte et qui comprend un partenaire de liaison spécifique à l'analyte,
(iii) un troisième domaine qui est adapté pour une détection optique de l'analyte,
(iv) un quatrième domaine qui est adapté pour l'absorption d'excès d'agent d'élution,
(v) éventuellement un boitier
(b) recevoir un échantillon contenant l'analyte d'une surface d'échantillon avec un élément de frottement,
(c) mettre en contact l'élément de frottement avec le deuxième domaine de l'élément de test pendant une période d'au moins 10 secondes, pendant laquelle une partie de l'échantillon est transférée de l'élément de frottement à l'élément de test et une incubation de l'analyte avec le partenaire de liaison spécifique à l'analyte se produit,
(d) mettre en contact le premier domaine de l'élément de test incubé avec un agent d'élution, et
(e) déterminer la présence ou/et la quantité d'analyte,
dans lequel l'élément de test et/ou l'élément de frottement comprend un réactif de transfert d'analyte qui contient au moins une substance spécifique à l'analyte choisie parmi le groupe consistant en une protéine, un mélange de protéines, un carbohydrate et un alcool de sucre.

2. Procédé selon la revendication 1, **caractérisé en ce qu'**une bandelette de test chromatographique est utilisée en tant qu'élément de test.

3. Procédé selon la revendication 1 ou 2, **caractérisé en ce qu'**un anticorps ou un fragment fonctionnel d'anticorps est utilisé en tant que partenaire de liaison spécifique à l'analyte.

4. Procédé selon l'une des revendications 1 à 3, **caractérisé en ce que** un élément de frottement avec une ou plusieurs surfaces de frottement est utilisé, dans lequel l'élément de frottement et l'élément de test sont mis en contact l'un avec l'autre en particulier pour une durée d'environ 10 secondes à environ 600 secondes, ou d'environ 60 secondes à environ 180 secondes.

5. Procédé selon l'une des revendications 1 à 4, **caractérisé en ce que** l'élément de test comprend un réactif de transfert d'analyte, qui contient au moins une substance spécifique à l'analyte choisie parmi le groupe consistant en un carbohydrate et un sucre d'alcool, et/ou l'élément de frottement comprend un réactif de transfert d'analyte, qui contient au moins une substance spécifique à l'analyte choisie parmi le groupe consistant en une protéine et un mélange de protéines.

6. Procédé selon l'une des revendications 1 à 5, **caractérisé en ce que** le deuxième domaine de l'élément de test comprend de plus au moins un agent pour la préparation chimique et/ou mécanique de l'échantillon contenant l'analyte, dans lequel l'agent pour la préparation chimique de l'échantillon comprend en particulier une substance choisie parmi le groupe consistant en un acide, une base, un tampon, un solvant organique et un détergent, et dans lequel l'agent pour la préparation mécanique de l'échantillon comprend en particulier un tissu et/ou un non-tissé.

7. Procédé selon l'une des revendications 1 à 6, **caractérisé en ce qu'**il comprend une combinaison d'un format de test compétitif et d'un format de test non-compétitif.

8. Procédé selon l'une des revendications 1 à 7, **caractérisé en ce que** l'analyte est déterminé avec une spécificité d'au moins 95% et/ou avec une sensibilité d'au moins 90%.

9. Procédé selon l'une des revendications 1 à 8, **caractérisé en ce qu'**un stupéfiant, en particulier une substance choisie parmi le groupe consistant en les hallucinogènes dissociatifs, les hallucinogènes délirants, les empathogènes, les entactogènes, les hypnotiques, les narcotiques, les psychédéliques, les sédatifs et stimulants, est déterminé en tant qu'analyte.

10. Procédé selon l'une des revendications 1 à 9, **caractérisé en ce que** plusieurs analytes, en particulier 2 à 20 analytes différents, sont déterminés en même temps.

11. Procédé selon l'une des revendications 1 à 10, **caractérisé en ce qu'**un fluide corporel, en particulier du sang, de l'urine ou de la salive est utilisé en tant qu'échantillon.

12. Procédé selon l'une des revendications 1 à 11, **caractérisé en ce qu'**un échantillon avec un volume d'environ 0,05 µl à environ 20 µl, en particulier avec un volume d'environ 0,1 µl à environ 5 µl est utilisé.

13. Elément de test pour la détermination d'un analyte comprenant :
(i) un premier domaine qui est adapté pour recevoir l'agent d'élution,
(ii) un deuxième domaine qui est adapté pour l'application d'un échantillon contenant l'analyte et qui comprend un partenaire de liaison spécifique à l'analyte,
(iii) un troisième domaine qui est adapté pour la détection visuelle de l'analyte,
(iv) un quatrième domaine qui est adapté pour recevoir l'agent d'élution en excès,
(v) éventuellement un boitier, et
(vi) un réactif de transfert d'analyte qui contient au moins une substance spécifique à l'analyte choisie parmi le groupe consistant en une protéine, un mélange de protéines, un carbohydrate et un alcool de sucre.

14. Utilisation d'un élément de frottement dans un procédé selon l'une des revendications 1 à 12 pour recevoir un analyte d'une surface et pour transférer l'analyte à l'élément de test, qui contient au moins une substance de transfert d'analyte choisie parmi le groupe consistant en une protéine, un mélange de protéines, un carbohydrate et un alcool de sucre.

15. Kit pour la détermination d'un analyte comprenant :
(a) un élément de test comprenant
(i) un premier domaine qui est prévu pour recevoir l'agent d'élution,
(ii) un deuxième domaine qui est prévu pour l'application d'un échantillon contenant l'analyte et qui comprend un partenaire de liaison spécifique à l'analyte,
(iii) un troisième domaine qui est prévu pour la détection visuelle de l'analyte,
(iv) un quatrième domaine qui est prévu pour recevoir l'agent d'élution en excès, et
(v) optionnellement un boitier,
(b) un élément de frottement qui est prévu pour recevoir un échantillon de l'analyte,
dans lequel l'élément de test et/ou l'élément de frottement comprend un réactif de transfert d'analyte, qui contient au moins une substance de transfert d'analyte choisie parmi le groupe consistant en une protéine, un mélange de protéines, un carbohydrate et un alcool de sucre.
